(19) **Europäisches Patentamt**
**European Patent Office**
**Office européen des brevets**

(11) **EP 3 294 121 B1**

(12) **FASCICULE DE BREVET EUROPEEN**

(45) Date de publication et mention de la délivrance du brevet:
**03.09.2025 Bulletin 2025/36**

(21) Numéro de dépôt: **16731211.5**

(22) Date de dépôt: **13.05.2016**

(51) Classification Internationale des Brevets (IPC):
***A61B 5/021*** (2006.01)

(52) Classification Coopérative des Brevets (CPC):
**A61B 5/02125; A61B 5/02133; A61B 5/318; A61B 5/349; A61B 5/35; A61B 7/045**

(86) Numéro de dépôt international:
**PCT/FR2016/051138**

(87) Numéro de publication internationale:
**WO 2016/181087 (17.11.2016 Gazette 2016/46)**

(54) **PROCEDE ET DISPOSITIF DE DETERMINATION DE PARAMETRES REPRESENTATIFS D'UNE ACTIVITE CARDIOVASCULAIRE**

**VERFAHREN UND VORRICHTUNG ZUR BESTIMMUNG DER CHARAKTERISTISCHEN PARAMETER EINER KARDIOVASKULÄREN AKTIVITÄT**

**METHOD AND DEVICE FOR DETERMINING PARAMETERS REPRESENTATIVE OF A CARDIOVASCULAR ACTIVITY**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.05.2015 FR 1554342**

(43) Date de publication de la demande:
**21.03.2018 Bulletin 2018/12**

(73) Titulaire: **Cardiags**
**69008 Lyon (FR)**

(72) Inventeurs:
• **ZEGADI, Nacira**
 **69007 Lyon (FR)**
• **ZEGADI, Lakhdar**
 **69007 Lyon (FR)**
• **BEYA, Ouadi**
 **71200 Le Creusot (FR)**

• **HICHRI, Echrak**
 **3012 Berne (CH)**

(74) Mandataire: **IXAS Conseil**
 **22, avenue René Cassin**
 **69009 Lyon (FR)**

(56) Documents cités:
 **FR-A1- 2 973 998       US-A1- 2003 220 577**
 **US-A1- 2012 179 053    US-A1- 2015 265 163**
 **US-A1- 2016 066 801**

• **O BEYA ET AL: "EDA, approche non linéaire de débruitage des signaux cardiaques", CORESA 2013 - 16ÈME ÉDITION DU COLLOQUE COMPRESSION ET REPRÉSENTATION DES SIGNAUX AUDIOVISUELS, 29 November 2013 (2013-11-29), XP055296424**

**Description**

**Domaine technique de l'invention**

La présente invention concerne la surveillance ou le suivi médical à distance de l'état physiologique d'un individu et, plus généralement, la prévention et la détection précoce de problèmes cardio-vasculaires. Plus précisément, la présente invention concerne un dispositif d'aide au diagnostic et un procédé d'auscultation cardio-vasculaire d'un patient.

**Etat de la technique**

Pour surveiller ou assurer un suivi de l'état physiologique d'un patient, il est connu d'utiliser des enregistreurs, qui une fois mis en place sur le patient, permettent d'acquérir des signaux biologiques de type analogique ou juste une ou plusieurs de leurs caractéristiques, telles que le rythme cardiaque pour un électrocardiogramme (modalité électrique), la mesure d'une tension ou pression artérielle (modalité pression) ou l'écoute de bruits cardiaques (modalité mécanique du cœur).

L'examen clinique soigneux d'un patient atteint de cardiopathie est une méthode qui apporte souvent des informations importantes quant à l'état du système cardiovasculaire au moment de l'examen. L'examen cardiovasculaire porte sur l'appréciation soigneuse des pouls artériels et des pouls veineux, la palpation systématique de la pointe du cœur et une auscultation cardiaque attentive. La compréhension des événements consécutifs du cycle cardiaque est indispensable à la conduite d'un examen cardiovasculaire précis.

L'auscultation - c'est-à-dire l'écoute des sons internes du coeur - constitue la technique la plus communément employée pour l'évaluation de l'état de santé d'un individu. Le médecin se concentre d'abord sur l'écoute des bruits cardiaques en commençant par un premier bruit. Après deux bruits physiologiques à différents points d'auscultation (appelés B1 et B2), le médecin cherche d'éventuels bruits supplémentaires (B3 et B4), avant de se concentrer sur les souffles.

L'auscultation d'organes tels que le cœur, les vaisseaux sanguins et/ou les poumons, ainsi que l'interprétation des sons produits par ces organes sont des outils fondamentaux dans la détection de maladies des systèmes cardio-vasculaires et pulmonaires. Les médecins généralistes utilisent aujourd'hui plusieurs appareils individuellement pour détecter une anomalie cardio-vasculaire ou pulmonaire :

- le stéthoscope pour écouter les sons produits par le cœur, les vaisseaux sanguins et/ou les poumons,
- le tensiomètre pour mesurer une pression artérielle.

Pour une évaluation plus approfondie de certains paramètres, il faut recourir à des méthodes d'investigation invasives ou des méthodes non invasives, telles que l'électrocardiographe, l'échocardiographie ou la radiologie. Ces dernières sont tout aussi coûteuses en temps et en moyens, et sont utilisables exclusivement par un personnel spécialisé et dans des locaux spécialisés (hôpitaux et cabinets de spécialistes).

Un inconvénient du stéthoscope est qu'il nécessite des années d'apprentissage pour une interprétation correcte des sons qu'il permet d'écouter. Un inconvénient de l'échocardiographie est qu'il s'agit d'un matériel non ambulatoire lourd et encombrant. L'acte en lui même est long, coûteux et nécessite un opérateur expérimenté. L'électrographie douze dérivations est un appareil qui fait une analyse précise de la modalité électrique. Ces deux appareils, largement utilisés par les cardiologues, sont des appareils de diagnostic et ne sont pas adaptés au dépistage précoce et à la prévention.

Aujourd'hui, l'éclatement des maladies chroniques rajouté à la problématique du vieillissement de la population, avec ses pathologies associées, a entrainé un déséquilibre entre le nombre de patients à suivre et le nombre de spécialistes qui ne cesse de diminuer. Le patient d'aujourd'hui est demandeur d'autonomie, de mobilité, de sorte que les notions de soins autonomes et de « n'importe où, n'importe quand » sont de plus en plus demandées.

Des actes de télémédecine sont recherchés en particulier dans les zones où manquent des spécialistes, des actes médicaux sont ainsi faits par des non spécialistes et l'interprétation assurée par des spécialistes distants. Les autorités de santé encouragent aussi les actes de prévention faits par des acteurs de proximité, tels que le médecin généraliste des réseaux de santé ou des centres médicaux. Le recours aux spécialistes distants permet que ceux-ci optimisent leur temps et est possible grâce aux technologies de l'information et de la communication et aux multiples outils disponibles pour la transmission, la sécurisation, l'accès et le traitement des données, tels que les ordinateurs personnels (PC), les tablettes ou encore les téléphones intelligents.

Afin de remédier aux inconvénients listés immédiatement ci-dessus, FR-A-2 973 998 a proposé un procédé permettant au praticien de détecter une anomalie cardiaque et/ou pulmonaire de manière rapide, simple et efficace, en croisant dans le même cycle cardiaque des données synchrones ou modalités complètes et complémentaires caractérisant l'activité cardio-vasculaire et/ou pulmonaire tel que le ferait l'expert, afin de fournir une aide au diagnostic au non expert. Ces données sont acquises par un seul appareil qui intègre des capteurs modernes spécifiques, adaptés aux besoins cités ci-dessus. L'appareil recueille des signaux qui sont de nature similaire à celles acquises par les différents instruments monomodaux, i.e. stéthoscope, tensiomètre, électrocardiogramme ou ECG, le tensiomètre ne donnant que 2 points de la courbe hémodynamique (maximale systolique, minimale diastolique).

Ce procédé prévoit une acquisition multimodale synchrone, à savoir une première acquisition d'une courbe acoustique cardiaque et d'une courbe acoustique pulmonaire, une acquisition d'une courbe de pression ainsi qu'une acquisition d'une courbe électrique cardiaque. Il y a ensuite mise en correspondance de ces différentes courbes, en vue d'estimer des paramètres représentatifs de l'activité cardiaque et/ou pulmonaire. La courbe électrique, ou électrocardiogramme (ECG), constitue une référence temporelle dans le cycle cardiaque pour tous les signaux acquis simultanément.

La présente invention vise tout d'abord à améliorer l'enseignement de FR-A-2 973 998, en particulier en fournissant des données supplémentaires au praticien.

Par ailleurs, de nombreux travaux récents ont concerné la pression sanguine sur différents sites du corps humain. Des méthodes non invasives ont été développées pour acquérir des ondes dont la chronologie et la morphologie correspondent à celles des pressions intra cardiaques, indépendamment du niveau de ces pressions. Des méthodes ont été développées pour l'analyse intrinsèque de la morphologie de la courbe de la pression artérielle et pour la détermination de certains paramètres à partir de cette courbe.

L'artère carotide est considérée comme un bon reflet des artères centrales et en particulier de la racine de l'aorte. Cependant, la pression artérielle systolique, notamment, change de façon significative au niveau des artères périphériques. De nombreuses études démontrent que les pressions centrales, systolique ou pulsée, sont davantage prédictives d'évènements cardio-vasculaires que la pression mesurée au bras. D'autres études récentes montrent que, pour un même abaissement de la pression artérielle périphérique, des stratégies thérapeutiques différentes peuvent ne pas avoir le même impact sur la pression systolique centrale, et que cela peut être important sur le plan de la prévention des complications cardiovasculaires.

Pour l'évaluation de la pression du pouls carotidien (ou pression centrale), on connaît tout d'abord une première méthode. Cette dernière prévoit d'acquérir une onde qui reflète les déplacements de la paroi artérielle pendant le passage de l'onde par des capteurs mécaniques (de type piézoélectrique, pour le dispositif connu sous la référence commerciale « COMPLIOR »). La courbe obtenue est appelée un mécanogramme.

On connait aussi une autre méthode, dite tonométrie, qui est alternative à la première méthode immédiatement décrite ci-dessus. La tonométrie, qui est très sensible à la position et à l'angle du capteur, permet d'accéder à la même grandeur, soit directement sur le site carotidien, ou indirectement en appliquant à la courbe de pression radiale une analyse basée sur l'application d'une « fonction de transfert ». Le dispositif connu sous la référence commerciale « SPHYGMOCOR » opère par tonométrie. La justification de cette dernière a été mise en doute et des travaux récents, en cours, cherchent à s'affranchir de ce problème.

On connait aussi une méthode électro-optique dite « photopléthysmographie », pour la mesure de la pulsation artérielle. Celle-ci est très sensible aux bruits environnants: lumière ambiante et mouvements volontaires ou involontaires du sujet. La courbe de pression centrale est déduite par application de la fonction de transfert citée précédemment avec les mêmes réserves. Certains ont gardé des manchons gonflables comme pour la mesure au tensiomètre brassard, soit une méthode oscillométrique.

De nombreuses études ont montré un intérêt grandissant à l'utilisation de la rigidité artérielle comme nouveau facteur prédictif pour la mortalité toutes causes confondues, notamment la mortalité cardiovasculaire, la cardiopathie ischémique et les accidents vasculaires cérébraux. Les mesures de pression systolique ou pulsée, carotidienne ou aortique, et de l'index d'augmentation ne sont que des estimations indirectes de la rigidité artérielle, alors que la vitesse de l'onde de pouls est une mesure directe de la rigidité artérielle. Cependant, l'utilisation en pratique clinique de la vitesse d'onde de pouls (ou VOP) reste limitée, en absence d'homogénéité des méthodes et par manque de valeurs de référence.

FR 3 005 256 décrit un procédé de calcul de cette vitesse d'onde de pouls, dans lequel on détermine tout d'abord les instants respectifs d'arrivée de cette onde en deux endroits distincts du corps, en l'occurrence l'orteil et le doigt. On calcule ensuite la durée de transit aortique, à partir de la différence entre ces instants d'arrivée. Enfin, on accède à la vitesse d'onde de pouls proprement dite, à partir de cette durée de transit. Malgré la simplicité d'accès aux sites de mesure qu'il propose, ce procédé implique certains inconvénients, liés en particulier au choix des sites de mesure utilisés. C'est seulement la démonstration d'une valeur prédictive pour la survenue des complications cardio-vasculaires qui permettra de garantir que les nouveaux appareils de mesure de rigidité artérielle sont valides.

Des méthodes telles que l'IRM (Imagerie à Résonance Magnétique), l'échocardiographie et la tonométrie sont complexes et coûteuses.

A cet égard, EP 1 338 242 a pour objet un procédé de détermination de la vitesse d'onde de pouls, dans lequel on acquiert notamment une onde de pression artérielle au niveau de la carotide du patient. Cette acquisition est mise en œuvre par une technique tonométrique, laquelle souffre de plusieurs inconvénients qui freinent son implantation en milieu hospitalier, dans le cadre d'examens en routine clinique. Bien que d'une simplicité apparente, la nécessité d'une bonne expérience de l'opérateur couplée à la qualité du capteur de pression est indispensable afin d'obtenir une mesure fiable et précise de la vitesse de l'onde de pouls.

Un document de consensus (Laurent S, Cockcroft J, Van Bortel L, Boutouyrie P, Giannattasio C, Hayoz D, Pannier B, Vlachopoulos C, Wilkinson I,Struijker-Boudier H; European Network for Non-invasive Investigation of Large Arteries. Expert consensus document on arterial stiffness: methodological issues and clinical applications. Eur Heart J. 2006 ; 27:2588-605) a défini la vitesse de l'onde de pouls carotido-fémorale (VOPcf) comme l'étalon or de la mesure de la rigidité artérielle, car il s'agit d'une mesure non-invasive simple, robuste et répétable. Par ailleurs, un nombre important d'études épidémiologiques ont démontré son caractère prédictif pour de nombreux évènements incluant la mortalité totale, la mortalité cardio-vasculaire, la survenue d'évènements coronaires et d'accidents vasculaires cérébraux (AVC), la récupération fonctionnelle après AVC, la mise en hémodialyse et la survenue d'une hypertension artérielle. Ces résultats ont été confirmés par deux méta-analyses sur données publiées et sur données individuelles. La détermination de la rigidité aortique par la mesure de la (VOPcf, carotido-fémorale) a été inscrite dès 2007 sur la liste des mesures qui devraient être réalisées chez l'hypertendu pour détecter une atteinte des organes cibles. Les recommandations 2013 de la Société Européenne d'Hypertension (ESH) et de la Société Européenne de Cardiologie (ESC) positionnent la mesure de la VOPcf en classe IIa (« poids de l'évidence/opinion en faveur de l'efficacité/utilité ») avec un niveau de preuve « B ». (http://www.escardio.org/guidelines-surveys/esc-guidelines/Documents).

C'est la VOP carotido-fémorale qui est la plus populaire en France, et dans la majeure partie du monde. La VOP bras cheville a été développée en Asie pour éviter d'exposer la région de l'aine. La réalité anatomique du trajet parcouru par l'onde de pouls n'est toujours pas déterminée. Certains appareils plus anciens comme COMPLIOR et SPHYGMOCOR décrits ci-dessus, ou encore « PULSEPEN », ont démontré leur valeur prédictive pour les évènements cardio-vasculaires.

Les cardiologues occupent l'essentiel de leur temps à poser des diagnostics, mais les tâches de suivi des malades dont le diagnostic est déjà établi, y compris celles qui concernent des dispositifs invasifs posés aux patients, sont chronophages. Le médecin généraliste prend le relais pour les tâches de prévention et de suivi, mais il ne dispose pas d'outils simples et adaptés à ces tâches.

La VOP carotido-fémorale, qui est la plus utilisée, prend en compte plusieurs segments artériels où l'onde de pouls se propage selon des sens opposés. Toutes les méthodes de correction pour compenser les trajets opposés sur un court segment se sont avérées induire des causes supplémentaires d'approximation. La réalité anatomique du trajet parcouru par l'onde de pouls n'est toujours pas déterminée. Les dispositifs utilisant un recalage du temps de transit sur l'ECG dépendent de la variation du cycle cardiaque en particulier en cas de patients avec problèmes de rythme, où la VOP n'est donc pas mesurable.

La mesure de la longueur parcourue par l'onde de pression est un point faible de la mesure non-invasive de la rigidité artérielle par la VOP, en particulier pour la vitesse de pouls carotido-fémorale. En effet, il est nécessaire d'estimer la longueur parcourue par l'onde de pression entre les deux sites de mesure. La mesure de la distance entre les deux sites anatomiques n'est pas bien définie et est encore sujette à controverse :

- Distance directe entre le site de mesure carotide et celui fémoral qui surestime la VOP
- Distance précédente avec soustraction de la distance carotide-fourchette sternale.
- Enfin, la différence entre la distance fourchette sternale-fémorale et la distance carotide-fourchette sternale a tendance à être prise par défaut, afin d'éliminer le segment artériel crosse aortique-carotide dans la mesure de la rigidité aortique.

Actuellement les recommandations européennes préconisent de considérer pour la distance les 80% de la distance directe entre le site carotidien et le site fémoral pour proposer un standard de mesure et un seuil de VOP anormale qui tiendrait compte des différences entre les VOP mesurées par des dispositifs de mesure différents. Cependant, le temps de transit de l'onde de pouls est resté identique. Cette correction de la distance seule, permet quant à elle de ne prendre en compte que le segment aortique.

Compte tenu de ce qui précède, un objectif de la présente invention est de remédier, au moins partiellement, aux inconvénients de l'art antérieur évoqués ci-dessus.

Un autre objectif de l'invention est de proposer un procédé permettant de déterminer, de façon simple et rapide, des paramètres représentatifs d'une activité cardiovasculaire, notamment la vitesse d'onde de pouls.

Un autre objectif de l'invention est de proposer un tel procédé de détermination, qui met en oeuvre l'acquisition d'une courbe de pression artérielle, de type mécanogramme, et qui s'affranchit donc des lacunes de la tonométrie.

Un autre objectif de l'invention est de proposer un procédé permettant de déterminer les paramètres précités, de façon fiable, en mettant simultanément en relation les paramètres cardiaques et vasculaires.

Un autre objectif de l'invention est de permettre le calcul de paramètres à partir de points ou repères initiaux, précis, déterminés et en tenant compte de l'origine de ces ondes (soit au niveau du cœur) et de la chronologie des événements dans le cycle cardiaque.

Un autre objectif de l'invention est de proposer un procédé permettant de déterminer les paramètres précités, grâce à des moyens de traitement adaptés à l'usage des praticiens.

## Objets de l'invention

Selon l'invention, les objectifs ci-dessus sont atteints au moyen d'un procédé de détermination d'au moins un paramètre représentatif d'une activité cardiovasculaire, comprenant les étapes suivantes :

- acquisition d'une courbe représentative d'une onde acoustique cardiaque, ou courbe acoustique
- acquisition d'une courbe de type mécanogramme, représentative d'une onde de pression sanguine, ou courbe de pression, par un capteur de pression adapté pour mesurer des vibrations d'une fréquence inférieure à 5 Hz
- acquisition d'une courbe représentative d'une onde électrique cardiaque, ou courbe électrique,
- mise en correspondance de la courbe acoustique, de la courbe de pression et de la courbe électrique pour estimer au moins un paramètre représentatif d'une activité cardiovasculaire,
- affichage du ou des paramètre(s) déterminé(s),

au moins un paramètre représentatif étant une durée représentative d'une activité cardiovasculaire,
procédé dans lequel on mesure cette durée en prenant, en tant que début de cette durée, l'un parmi un point de la courbe acoustique et un point de la courbe de pression et, en tant que fin de cette durée, l'autre parmi le point de la courbe acoustique et le point de la courbe de pression, ladite durée étend calculée à partir de deux points mesurés sur deux courbes de morphologies différentes, à savoir la courbe acoustique et la courbe de pression,
cette mesure permettant de déterminer la durée de transit correspondant à la durée de transit entre l'endroit où est acquise l'onde acoustique et l'endroit où est acquise l'onde de pression artérielle, et
procédé dans lequel on recale des points caractéristiques liés à des événements physiologiques cardiaques qui leur ont donné naissance, sur le même axe temporel, ce qui permet d'avoir une empreinte cardio-vasculaire de l'individu ou réglette numérique cardiaque.

L'invention se distingue clairement des enseignements antérieurs, tels que décrits respectivement dans FR 2 973 998 et EP 1 338 242.

Tout d'abord, l'invention inclut l'acquisition d'une courbe de type mécanogramme, qui ne peut pas être obtenue par mise en œuvre de la tonométrie, objet de EP 1 338 242. On rappellera qu'un mécanogramme est une courbe obtenue par l'enregistrement des mouvements ou des phénomènes vibratoires nés de l'activité mécanique d'une partie du corps, notamment du coeur. La technique précitée de tonométrie nécessite, de manière générale, un utilisateur très aguerri. EP 1 338 242 introduit une notion de réglage supplémentaire, qui va dans ce même sens, à savoir de faire appel à un praticien expérimenté. L'objectif de l'invention est d'avoir un dispositif dont au moins l'acquisition des mesures peut être faite par un non spécialiste, l'interprétation des mesures et le diagnostic pouvant rester du ressort du spécialiste. Cependant l'analyse automatique des courbes, qui peut alerter le non spécialiste, tel que le médecin généraliste, et peut être donc à l'origine d'un dépistage précoce.

Par ailleurs, selon l'invention, la durée représentative est obtenue en prenant un point dit « de début » et un point dit « de fin ». Le point de début est pris sur une première courbe (PCG ou CAR), alors que le point de fin est pris sur l'autre courbe (respectivement CAR ou PCG). Ces deux points sont donc pris sur deux courbes différentes. On peut donc en conclure que l'invention se distingue de l'enseignement de FR 2 973 998. En effet, aux lignes 5 à 15 de la page 18, ce dernier document décrit que ces deux points sont pris sur une même courbe, en l'occurrence le PCG.

Selon d'autres caractéristiques de l'invention :

a) on accède à la vitesse d'onde de pouls (VOP) à partir de la durée de transit, en utilisant la formule : VOP = D / A2-I, où D est la distance entre l'endroit où est acquise l'onde acoustique et l'endroit où est acquise l'onde de pression artérielle (transit de l'onde aorte ascendante-carotide).

b) on accède, également, à la vitesse d'onde de pouls en localisant le pied de l'onde de pouls de la courbe de pression et en déterminant la durée entre ce pied d'onde et un repère initial matérialisant l'ouverture de la valve sigmoïdienne.

c) on accède à au moins une autre durée représentative d'une activité cardiaque, et on mesure cette autre durée en prenant, en tant que début de cette autre durée, l'un parmi un point de la courbe acoustique et un point de la courbe électrique et, en tant que fin de cette durée, l'autre parmi le point de la courbe acoustique et le point de la courbe électrique.

d) l'autre durée représentative d'une activité cardiaque est le temps de transformation, qui sépare le minimum de l'onde Q de la courbe électrique et le début du bruit B1 de la courbe acoustique, ou bien la durée de systole électromécanique, qui sépare le minimum de l'onde Q de la courbe électrique et le début de la composante A2 du bruit B2 de la courbe acoustique.

e) on accède à au moins une durée supplémentaire représentative d'une activité cardiaque, et on mesure cette durée supplémentaire en prenant, en tant que début et fin de cette autre durée, deux points appartenant à une même courbe.

f) on mesure au moins une durée complémentaire représentative d'une activité cardiaque, à partir de deux autres durées représentatives.

g) on réalise une droite d'interpolation d'une première partie de la courbe de pression, on accède au point de fermeture mitrale correspondant à l'intersection entre cette droite d'interpolation et une droite passant par le premier bruit de la courbe acoustique, on détermine la pente de cette droite d'interpolation, on réalise une droite d'interpolation d'une autre partie de la courbe de pression, on détermine le point d'ouverture mitrale et on accède à la durée de relaxation iso volumétrique, correspondant à la durée entre l'incisure catacrote de la courbe de pression et le point de fermeture mitrale.

h) On accède alors à un intervalle plus précis pour déterminer la présence des bruits pathologiques B3 et B4 (point OM et début de l'onde T de la courbe ECG)

i) on détecte l'onde T de la courbe électrique au moyen d'une méthode par morphologie mathématique multi échelles.

j) on utilise, pour cette méthode par morphologie mathématique multi échelles, trois éléments structurants, à savoir un premier élément triangulaire et deux autres éléments hexagonaux, ces éléments structurants présentant des tailles, des largeurs et des amplitudes prédéfinies.

k) on traite la courbe acoustique en utilisant une décomposition modale empirique, on obtient différentes courbes décomposées, dites d'intérêt, à partir de cette décomposition, et on sélectionne au moins une courbe décomposée préférée ayant une énergie de signal maximale.

l) on sélectionne la courbe décomposée préférée ayant une valeur minimale d'un critère d'arrêt : $Rj = 1 - [E(IMF^2 j) / E(s^2(k))]$, où $E(IMF^2 j)$ désigne l'énergie de chaque courbe décomposée obtenue par la décomposition modale empirique, pour j variant de 1 à n, n étant le nombre de courbes décomposées obtenues lors d'une décomposition modale empirique donnée, et où $E(s^2(k))$ désigne l'énergie globale du signal initial.

m) on garde uniquement les courbes décomposées d'intérêt qui appartiennent à une plage de fréquences prédéterminée.

n) on calcule un signal utile de la courbe acoustique, correspondant à la somme des courbes décomposées d'intérêt.

o) on localise les premier et deuxième bruits B1 et B2 de la courbe acoustique en synchronisant le signal utile avec la courbe électrique.

p) on localise le début du premier bruit B1 à la fin du pic R de la courbe électrique et on localise le début du deuxième bruit B2 à la fin du pic T de la courbe électrique.

q) on applique la transformée de Huang Hilbert au signal utile de la courbe acoustique, ce qui permet d'obtenir l'enveloppe modale de ce signal utile, on détecte les maxima de cette enveloppe modale, on réalise une opération de seuillage, de façon à localiser les premier et deuxième bruits B1 et B2 de la courbe acoustique, et on détecte la probabilité de présence d'un souffle systolique ou diastolique entre les bruits B1 et B2

r) on distingue, dans l'intervalle du bruit B1, la composante mitrale de la composante tricuspide et, dans l'intervalle du bruit B2, la composante aortique de la composante pulmonaire ; cette étape peut être mise en oeuvre par toute méthode mathématique appropriée, de type connu en soi ;

s) on recale des points caractéristiques liés à des évènements physiologiques cardiaques sur le même axe temporel, ce qui permet d'avoir une empreinte cardio-vasculaire de l'individu ou réglette numérique cardiaque.

t) on prend le repère initial OS-PCG de l'ouverture sigmoïdienne, ou pied de l'onde, sur la réglette numérique cardiaque.

u) on prend, en tant que repère initial, le point Q sur la réglette numérique cardiaque, et recalé à partir du complexe QRS de l'ECG.

Selon l'invention, on met en correspondance les trois courbes de types différents. La courbe électrique cardiaque, de type électrocardiogramme, constitue une base de temps pour les deux autres ondes. A partir de ces trois courbes, on peut déduire des paramètres représentatifs de l'activité cardiaque, des paramètres caractérisant les vaisseaux sanguins.

Le procédé de l'invention prévoit une acquisition multimodale synchrone, à savoir une première acquisition d'une onde acoustique cardiaque et d'une onde acoustique pulmonaire, une acquisition d'une (ou plusieurs) ondes de pression intracardiaque ou vasculaire, ainsi qu'une acquisition d'une onde électrique cardiaque. Il y a ensuite mise en correspondance de ces différentes ondes, en vue d'estimer des paramètres représentatifs de l'activité cardiaque et/ou pulmonaire et, simultanément, des paramètres caractérisant les vaisseaux sanguins. L'invention permet en particulier d'accéder à la vitesse de propagation de l'onde de pouls, encore dénommée Vitesse d'Onde de Pouls ou « VOP », qui permet d'apprécier la rigidité artérielle.

Le procédé conforme à l'invention est tout d'abord avantageux, en ce qu'il permet d'accéder à la valeur des paramètres précités, avec une meilleure précision. En effet, il est du mérite de la Demanderesse d'avoir identifié que cette précision est garantie, en prenant un point de l'onde acoustique, en tant que point de départ d'une durée caractéristique de l'activité cardiovasculaire.

Par ailleurs, l'invention autorise l'accès à de tels paramètres avec une rapidité en «quasi temps réel», compatible avec une utilisation efficace dans le cadre d'une brève consultation médicale. Les calculs sont effectués sur la courbe électrique, puis sur la courbe acoustique et enfin sur la courbe de pression avec un recalage sur un même outil de type « réglette numérique ». Des connaissances a priori sont rajoutées pour la précision des repères. Selon l'invention, ce temps de traitement et d'auscultation est réduit, de sorte que le médecin généraliste peut l'utiliser efficacement lors d'une consultation habituelle de 15 à 20 minutes de même qu'en mobilité.

De plus, les moyens de traitement utilisés dans le procédé conforme à l'invention demandent relativement peu de cycles de calculs. De la sorte, ils moins lourds que ceux mis en oeuvre dans l'art antérieur, ce qui les rend adaptés à une tablette ou à un téléphone intelligent.

Le procédé conforme à l'invention permet de faire du dépistage précoce, dans le cadre de la prévention ou du suivi. L'acte peut être fait par le médecin généraliste, mais l'interprétation et la pose du diagnostic reste du domaine du cardiologue.

Le dispositif conforme à l'invention peut être donc utilisé même par un non spécialiste tel que le médecin généraliste pour analyser un état cardiaque, vasculaire et/ou des poumons grâce à des courbes et des paramètres afin de prévenir et d'établir une détection précoce de certaines pathologies cardiovasculaires. Il peut alors envoyer rapidement un rapport documenté à l'expert, à distance, en quasi instantané, en cas d'anomalies.

Le spécialiste/cardiologue utilise ensuite des moyens plus ou moins sophistiqués dont il dispose usuellement, comme l'échographie, pour pousser ses investigations et poser un diagnostic. Ces échanges peuvent aussi se faire entre spécialistes pour un complément, une vérification ou un deuxième avis, ou encore avec un professionnel de santé tel qu'une infirmière. En effet l'ergonomie de l'appareil, ainsi que le choix et le conditionnement des capteurs, sont adaptés pour le permettre. L'analyse paramétrique est particulièrement adaptée au suivi. Une maladie comme l'insuffisance cardiaque nécessite un suivi continu, nécessite de grouper plusieurs signes lesquels comparés à des seuils permettent d'éviter des hospitalisations fréquentes, non programmées, mal vécues par le patient et coûteuses pour la collectivité.

Selon une caractéristique avantageuse de l'invention, de nouvelles méthodes de traitement sont utilisées sur la courbe électrique, puis sur la courbe acoustique et enfin sur la courbe de pression. Ceci permet de déterminer précisément la position de repères caractéristiques du cycle cardiaque, ramenés sur un seul et même axe temporel ayant comme origine temporelle le cœur (on parle de recalage temporel). Ces points initiaux permettront une évaluation simultanée de paramètres cardiaques et vasculaires dont les valeurs à un instant donné et/ou la comparaison à un instant ultérieur permettront la prévention et le suivi cardiaque et vasculaire d'un patient, ainsi que la détermination du profil cardiovasculaire d'un individu.

Un recalage de tous les points caractéristiques du cycle cardiaque est fait sur un axe unique (au moyen d'un outil qu'on peut dénommer « réglette numérique chronologique et quantitative »), permettant de déduire des paramètres cardiaques et dont au moins un point permettra de calculer des paramètres vasculaires au niveau de sites de mesure de pression sanguine. En effet, la courbe de pouls mesurée sur des sites autres que carotidien peut montrer une morphologie avec une incisure catacrote peu marquée, le calcul peut être fait aisément et simultanément sur le pied de l'onde de pouls. Pour les sites qui excluent le site cœur, la mesure peut réalisée en différé en déplaçant le capteur de pression d'un site à un autre avec un recalage sur la « réglette chronologique ».

La présente description divulgue également un procédé, qui ne fait pas partie de l'invention, en vue de la détermination d'au moins un paramètre représentatif d'une activité cardiovasculaire, comprenant les étapes suivantes :

- acquisition d'une courbe représentative d'une onde acoustique cardiaque, ou courbe acoustique
- acquisition d'une courbe de type mécanogramme, représentative d'une onde de pression sanguine, ou courbe de pression,
- acquisition d'une courbe représentative d'une onde électrique cardiaque, ou courbe électrique,
- mise en correspondance de la courbe acoustique, de la courbe de pression et de la courbe électrique pour estimer au moins un paramètre représentatif d'une activité cardiovasculaire,
- affichage du ou des paramètre(s) déterminé(s),

ledit procédé étant caractérisé en ce qu'au moins un paramètre représentatif est une durée représentative d'une activité cardiovasculaire.

Ce procédé de détermination peut comprendre toutes les caractéristiques a) à v) ci-dessus ou toute combinaison techniquement compatible de ces caractéristiques.

La présente description divulgue également un procédé, qui ne fait pas partie de l'invention en vue de la détection de l'onde T d'une courbe électrique cardiaque, de type électrocardiogramme, au moyen d'une méthode par morphologie mathématique multi échelles. Ce procédé de détection peut comprendre toutes les caractéristiques a) à v) ci-dessus ou toute combinaison techniquement compatible de ces caractéristiques.

La présente description divulgue également un procédé, qui ne fait pas partie de l'invention, en vue du traitement d'une courbe, notamment d'une courbe acoustique cardiaque, de type phonocardiogramme (PCG), dans lequel on utilise une décomposition modale empirique, on obtient différentes courbes décomposées, dites d'intérêt, à partir de cette décomposition, et on sélectionne au moins une courbe décomposée préférée ayant une énergie de signal maximale.

Ce procédé de traitement peut comprendre toutes les caractéristiques a) à v) ci-dessus ou toute combinaison techniquement compatible de ces caractéristiques.

L'invention a également pour objet un dispositif pour la détermination d'au moins un paramètre représentatif d'une activité cardiovasculaire, comprenant:

- au moins un capteur acoustique, pour l'acquisition de ladite courbe acoustique
- au moins un capteur de pression, adapté pour mesurer des vibrations d'une fréquence inférieure à 5 Hz, pour l'acquisition de ladite courbe de pression,
- au moins un capteur électrique, pour l'acquisition de ladite courbe électrique,
- des moyens de traitement,
- des moyens d'affichage du ou des paramètre(s) estimé(s) et tel que le dispositif soit configuré pour mettre en œuvre un procédé tel que ci-dessus.

Selon une caractéristique avantageuse, ce dispositif comprend en outre une lyre propre à être connectée audit capteur acoustique, ladite lyre possédant un microphone et des moyens de traitement du son, propres à acquérir au moins un enregistrement numérique d'une dictée reçue par le microphone.

Selon une caractéristique avantageuse, ce dispositif comprend en outre un capteur de pression différentiel, qui comprend un corps possédant un volume intérieur, un fond délimitant deux chambres dans ce corps, une première chambre étant à la pression atmosphérique et une deuxième chambre étant placée de manière à venir au contact de la peau d'un patient, ce capteur comprenant deux orifices de mesure de pression, un premier orifice débouchant dans la première chambre et un deuxième orifice débouchant dans la deuxième chambre.

Selon une caractéristique avantageuse, ce dispositif comprend en outre un module destiné à être posé sur le thorax d'un patient, ce module étant équipé d'un capteur central formant capteur acoustique, ainsi que de trois électrodes sèches réparties régulièrement à la périphérie de ce capteur central, formant capteurs électriques.

Selon une caractéristique avantageuse, il est prévu une dérivation bipolaire comme base de temps pour le PCG et pour calculer les paramètres cardiaques électriques, ainsi qu'une dérivation bipolaire de confirmation des résultats, ces deux dérivations étant prises par rapport au neutre, formée par la troisième électrode.

La présente description divulgue également un tel module thorax, ne faisant pas partie de l'invention, qui est pris de manière indépendante par rapport aux autres organes constitutifs du dispositif de détermination ci-dessus.

La présente description divulgue également une lyre médicale, qui ne fait pas partie de l'invention, propre à être connectée à un capteur acoustique, ledit capteur étant propre à l'acquisition d'une courbe représentative d'une onde acoustique cardiaque, ladite lyre possédant un microphone et des moyens de traitement du son, propres à acquérir au moins un enregistrement numérique d'une dictée reçue par le microphone.

La présente description divulgue également un capteur de pression différentiel, ne faisant pas partie de l'invention, pour l'acquisition de ladite courbe de pression, qui comprend un corps possédant un volume intérieur, un fond délimitant deux chambres dans ce corps, une première chambre étant à la pression atmosphérique et une deuxième chambre étant placée de manière à venir au contact de la peau d'un patient, ce capteur comprenant deux orifices de mesure de pression, un premier orifice débouchant dans la première chambre et un deuxième orifice débouchant dans la deuxième chambre.

Un des apports de l'invention est non seulement de développer des moyens ou procédés qui amènent une amélioration sur le plan acquisition ou sur le plan traitement des signaux, mais aussi de développer de nouvelles techniques qui répondent avec précision à des questions concernant les relations entre les signaux acquis et les phénomènes physiologiques qui les ont créés.

Un des apports de l'invention est de proposer un dispositif non invasif qui peut être utilisé en pratique quotidienne, pour des dépistages précoces de maladies cardiovasculaires. Les techniques échographiques ne peuvent pas être proposées dans le dépistage systématique en vue du diagnostic des maladies cardiovasculaires, du fait de leur coût et de la durée de chaque examen. De plus, elles requièrent un haut niveau d'expertise de l'opérateur, ce qui nous éloigne considérablement d'une évaluation en routine clinique.

Un autre apport de l'invention est de proposer en routine clinique l'évaluation d'un risque cardio-vasculaire global. De nombreux facteurs de risque cardio-vasculaires ont fait l'objet de nombreuses études. Le score de Framingham, score le plus utilisé dans le monde évalue le risque coronarien (événements mortels ou non) à 10 ans. A l'issue de ce calcul, doivent être considérés comme à haut risque cardiovasculaire (ou « HRCV ») les sujets dont le score est supérieur à 20 %. Cependant il reste très difficile d'évaluer le risque à l'échelle individuelle, puisque des individus identifiés à faible risque cardio-vasculaires subissent également ces évènements. Il est important d'intégrer d'autres facteurs de risque propres à un individu dans un risque cardio-vasculaire global. En effet, il a été démontré dans de nombreuses études épidémiologiques que la vitesse de l'onde de pouls (ou « VOP ») est un prédicteur indépendant de risque cardiovasculaire et de mortalité cardiovasculaire. La VOP apporte une information complémentaire et additive au risque de Framingham.

La rigidité des gros troncs artériels est la conséquence majeure du vieillissement artériel et comporte des modifications de l'organisation structurale des différents composants de la paroi artérielle. Le développement de la rigidité artérielle se manifeste par une accélération de la vélocité de l'onde du pouls (VOP), une élévation de la pression artérielle systolique PAS et de la pression pulsée PP et une baisse de la pression artérielle diastolique PAD. Ces manifestations observées au cours du vieillissement (âge chronologique) sont plus marquées chez les sujets avec un vieillissement artériel accéléré. Des facteurs d'accélération sont principalement l'HTA ; le diabète, l'insuffisance rénale, la fréquence cardiaque.

La VOP carotido-fémorale, est la plus utilisée. La mesure de la longueur parcourue par l'onde de pression est le maillon faible de la mesure non-invasive de la rigidité artérielle par la VOP, particulièrement sensible pour la vitesse de pouls carotido-fémorale. En effet, il est nécessaire d'estimer la longueur parcourue par l'onde de pression entre les deux sites de mesure. Cette grandeur prend en compte plusieurs segments artériels où l'onde de pouls se propage dans des sens opposés. Toutes les méthodes de correction pour compenser les trajets opposés sur un court segment se sont avérées induire des causes supplémentaires d'approximation.

Beaucoup de travaux antérieurs ont concerné l'usage d'appareils destinés à quantifier unilatéralement la VOP et d'autres paramètres caractérisant la pression artérielle (tels que la pression pulsée centrale, l'index d'augmentation...). L'onde qui est ainsi quantifiée a pour origine ou source l'éjection par le cœur d'un flux sanguin. Les deux phénomènes ne sont pas séparés : l'un est à l'origine de l'autre et tous deux sont influencés par l'état cardiaque et artériel à un instant donné de la vie d'un individu. Le procédé et le dispositif de l'invention permettent d'aligner des informations qui remontent d'autres corrélations, par exemple celle liant la contraction isovolumétrique (CI), elle-même reliée à la contractilité du cœur, aux paramètres qui caractérisent l'élasticité des artères. Pour l'évaluation d'un risque global rattaché à un état physiologique cardiaque et artériel de l'individu, il est particulièrement avantageux de s'appuyer sur des paramètres qui sont indépendants de la largeur du cycle cardiaque, en particulier de la fréquence cardiaque, tels que l'intervalle CI.

L'acquisition du phonocardiogramme (PCG) est liée au stéthoscope. Celui-ci a été qualifié de « subjectif » dans l'état de la technique actuel. En effet, quand on introduit la notion de bruits cardiaques B1, B2, présents à l'écoute ou visuellement, on qualifie des évènements qui durent en moyenne 100 ms. Dans la présente invention on s'intéresse à la notion de temps de transit de l'onde entre le cœur et la carotide et au recalage de ces points sur l'axe cardiaque. Ce temps est relativement court, typiquement moins de 30 ms. La synchronisation de l'ECG avec le PCG permet de distinguer immédiatement un bruit B1 d'un bruit B2 lorsqu'il y a un bon rapport signal sur bruit. Cependant ces bruits sont représentés par des traits épais dans les courbes représentatives du cycle cardiaque, ce qui confère une précision qui est insuffisante au regard de leur durée réelle et des oscillations qui les composent au regard de phénomènes plus courts, tels que le temps de transit.

Les connaissances divulguées dans FR 2 973 998 n'ont pas la précision suffisante pour évaluer le point de départ de l'onde et le point d'arrivée (ou décalé), sur un autre site artériel du corps. En effet le point recherché OA (pied de l'onde) sur la courbe de pression correspond à une égalité de pression ventriculaire et de pression aortique qui entraine l'ouverture de la valve aortique. Il faut trouver ce point précis dans l'intervalle B1 composé de plusieurs oscillations sur le PCG ; il en va de même pour le point FA, incisure catacrote correspondant à la fermeture aortique ou sigmoïdienne. La référence de B1 au pic R de l'ECG est très imprécise, dans EP 1 338 242. Dans FR 2 973 998, on utilise un fenêtrage par rapport au pic R puis une recherche du maximum de l'onde. Bien que plus précise que dans EP 1 338 242, cette méthode enseignée par FR 2 973 998 n'est pas totalement satisfaisante.

Conformément à l'invention, l'utilisation de la décomposition modale empirique (ou « EMD ») permet de choisir les échelles de décomposition utiles ou porteuses d'information. L'usage de l'enveloppe permet de délimiter les bruits B1, B2 et de préciser la présence de souffles. L'investigation dans ce travail est poussée, d'une part, sur le bruit B1 en distinguant sa composante mitrale de sa composante tricuspide et, d'autre part, sur le bruit B2 en distinguant sa composante aortique et sa composante pulmonaire. Cela est avantageusement permis grâce à la transformation en ondelettes puis en déterminant le début, la fin, le maximum et le minimum de chaque composante, pour les rattacher précisément aux phénomènes physiologiques réels se déroulant au niveau cardiaque.

L'interpolation permet de préciser les points critiques OM et OA suite à la détermination des sous intervalles B1 et B2. Par recalage de ces points sur un axe cardiaque ou réglette numérique cardiaque, cela permet d'accéder à des intervalles courts tels que les temps de la contraction isovolumétrique, de relaxation isovolumétrique, de transit de l'onde de pouls du cœur vers la carotide.

## Description des figures

L'invention va être décrite ci-après, en référence aux dessins annexés, donnés uniquement à titre d'exemples non limitatifs, dans lesquels :

- La figure 1 est une vue schématique, illustrant les différents composants d'un dispositif de détermination conforme à l'invention.
- La figure 2 est une vue schématique, illustrant la mise en oeuvre du dispositif de détermination conforme à l'invention.
- La figure 3a est un graphe illustre le profil d'éléments structurants conformes à l'invention, en vue du traitement de l'onde T de la courbe ECG.
- La figure 3b est un schéma blocs qui illustre le traitement de cette onde T au moyen de ces éléments structurants.
- La figure 4 illustre des courbes ECG et PCG brutes, destinées à être traitées selon l'invention.
- La figure 5 est une courbe, illustrant la variation de l'énergie de signal de plusieurs courbes décomposées, de type IMF, obtenues selon l'invention.
- Les figures 6a à 6c sont des courbes, illustrant le traitement de certaines de ces courbes décomposées.
- La figure 7 est une courbe de type PCG, obtenue après un traitement conforme à une variante de réalisation de l'invention.
- La figure 8 illustre des courbes ECG, CAR et PCG, mises en correspondance conformément à l'invention afin de déterminer des paramètres cardiovasculaires.
- La figure 9 illustre des courbes CAR et PCG, mises en correspondance conformément à l'invention afin de déterminer une valeur de relaxation iso volumétrique.
- La figure 10 est une courbe de type PCG, sur laquelle apparaissent des temps caractéristiques de l'activité cardiaque.
- La figure 11 illustre des courbes CAR et PCG, mises en correspondance conformément à l'invention afin de déterminer une vitesse d'onde de pouls.
- La figure 12 est une vue schématique de face, illustrant une lyre médicale conforme à l'invention.
- La figure 13 est une vue en coupe longitudinale, illustrant un capteur de pression conforme à l'invention.
- La figure 14 est une vue schématique, illustrant une réglette numérique utilisée dans le procédé conforme à l'invention.
- La figure 15 est une vue de face, illustrant un module conforme à l'invention, destiné à être posé sur le thorax d'un patient.

## Description détaillée

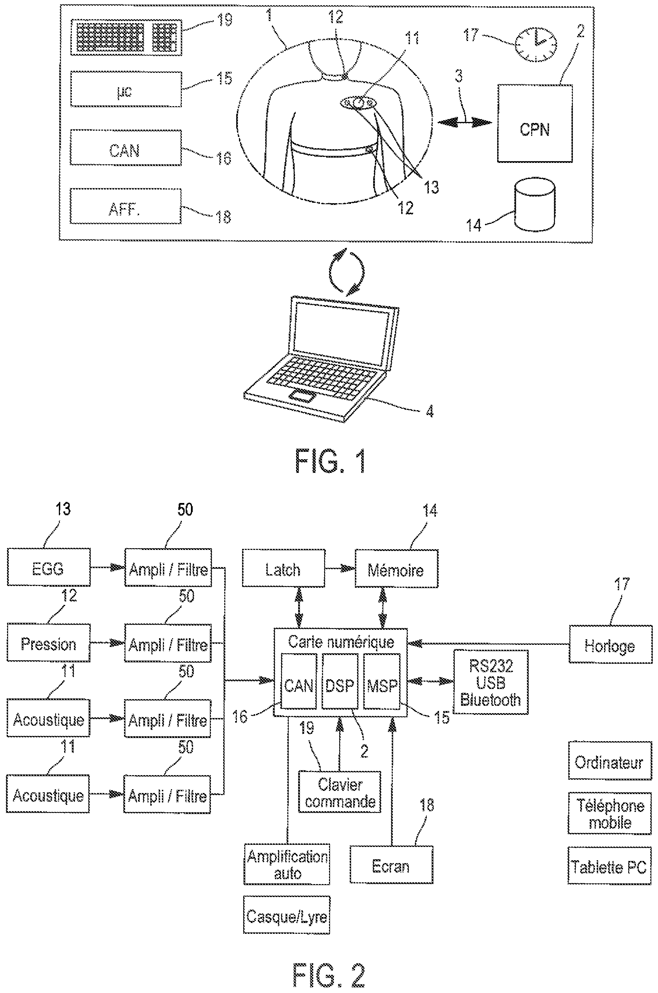

En référence à la figure 1, on a illustré un mode de réalisation du dispositif selon l'invention. Le dispositif comprend des moyens d'acquisition 1 et des moyens de traitement 2. Les moyens d'acquisition 1 permettent l'acquisition de différentes ondes pour déterminer l'état cardiaque d'un patient. Les moyens d'acquisition sont destinés à être positionnés sur le patient. Ces moyens d'acquisition peuvent être disposés sur une ceinture thoracique, et/ou une sangle ou tout autre support permettant un positionnement aisé et précis des moyens d'acquisition.

Les moyens d'acquisition 1 comportent :

- un (ou plusieurs) capteur(s) acoustique(s) 11, ou capteur(s) PCG
- un (ou plusieurs) capteur(s) de pression 12, ou capteur(s) CAR, et
- un (ou plusieurs) capteur électrique(s), ou capteur(s) ECG 13.

Ces différents capteurs permettent l'acquisition respectivement d'une onde de pression, d'une onde électrique, ainsi que d'une onde acoustique cardiaque ou pulmonaire. Ces informations complémentaires permettent de tenir compte en même temps de l'état mécanique du coeur, de l'état hémodynamique du coeur et de l'état électrique du coeur, ainsi que de l'état pulmonaire et du couplage entre ces aspects.

Les moyens de traitement 2 permettent de traiter les ondes enregistrées par les moyens d'acquisition 1. Les moyens de traitement peuvent être par exemple un processeur, un ordinateur personnel distant, un téléphone portable, une tablette moyens de traitement connu de l'homme du métier.

Les moyens de traitement 2 peuvent être intégrés ou distants des moyens d'acquisition 1. Dans tous les cas, les moyens d'acquisition 1 et de traitement 2 sont aptes à communiquer par l'intermédiaire de moyens de communication 3 avec ou sans fil. Les moyens de traitement 2 sont également aptes à communiquer avec un serveur distant 3 consultable par un praticien pour l'établissement d'un diagnostic.

Le dispositif comporte également :

- un convertisseur 16 analogique/numérique CAN pour numériser les ondes mesurées par les moyens d'acquisition,
- une horloge 17 pour l'indication de la date (jour, mois, année) et l'horaire de début (heure, minute, seconde) de chaque acquisition
- une mémoire 14 pour stocker les signaux numérisés, etc.
- des moyens de contrôle 15 pour commander l'acquisition des ondes par les trois capteurs 11, 12, 13.

Les moyens de contrôle 15 sont par exemple un microprocesseur, un microcontrôleur, ou tout autre dispositif connu de l'homme du métier. Le dispositif peut comprendre des moyens d'affichage 18, tels qu'un écran, et des moyens de saisie 19, tels qu'un clavier, pour permettre à l'utilisateur de sélectionner par exemple un type d'acquisition parmi les choix suivants :

- acquisition par le capteur acoustique seul, en position cardiaque
- acquisition par le capteur acoustique seul, en position pulmonaire
- acquisition par le capteur ECG seul,
- acquisition par le capteur de pression seul,
- acquisition simultanée par les capteurs acoustique et ECG
- acquisition par les capteurs de pression et ECG,
- acquisition par les capteurs de pression et acoustique,
- acquisition par les capteurs de pression, acoustique, ECG.

Le dispositif peut également comprendre des moyens de connexion, notamment pour la connexion d'un casque afin d'écouter les ondes durant leur acquisition, ou pour écouter les signaux numérisés traités par les moyens de traitement.

Avantageusement, les moyens de saisie permettent à l'utilisateur de choisir le signal numérique (i.e. de pression, ECG, acoustique), ainsi que la bande de fréquence (basse fréquence, moyenne fréquence ou haute fréquence, bande pulmonaire) qu'il souhaite écouter. L'écoute est restituée dans les mêmes conditions à distance.

A titre de variante, on peut utiliser un support différent d'une carte numérique, pour supporter les moyens de traitement 2, les moyens de contrôle 15 et le convertisseur 16. En particulier, on peut utiliser une carte mémoire amovible de stockage de données numériques. Dans ce cas, il s'agit typiquement d'une carte dite « SD » (sigle l'expression anglaise « *Secure Digital* »).

En termes structurels, les trois capteurs 11, 12 et 13 sont par exemple conformes à ceux, décrits dans FR-A-2 973 998. Ils sont donc représentés de façon schématique sur la figure 1 et ne seront pas décrits plus en détail dans ce qui suit.

Le capteur acoustique 11 est par exemple du type électret, dont la bande de fréquence couvre le domaine des vibrations sonores cardiaques. Ce capteur acoustique 11 présente typiquement une sensibilité de l'ordre du mV/pascal. Il comprend par exemple une chambre microphone, une coupelle et une membrane.

Le capteur de pression 12 permet l'acquisition d'une onde de pression artérielle. Ce capteur de pression est destiné à être positionné au niveau de la carotide, l'apex ou tout autre endroit sur le corps du patient. Les signaux recueillis par le capteur de pression 12 sont aussi des vibrations qui découlent de l'activité cardiaques. Ils diffèrent cependant des vibrations d'origine cardio-vasculaires par leur fréquence ultra-basse, inférieure à 5 Hz, leur intensité beaucoup plus forte et leur durée plus longue.

Conformément à l'invention, le capteur de pression 12, qui est de type mécanique, mesure les déplacements de la paroi artérielle pendant le passage de l'onde. La courbe obtenue, à l'issue de cette mesure, est dénommée un mécanogramme. Le principe de mesure appliquée dans le cadre de l'invention est donc fondamentalement différent d'autres méthodes connues, telles que par exemple la tonométrie.

Le capteur ECG 13 permet l'acquisition d'une onde électrique cardiaque. Il est destiné à être positionné sur le thorax du patient ou toute autre position du corps. Le capteur ECG peut comprendre une (ou plusieurs) électrode(s) montée(s) sur un support qui épouse la forme du thorax. Ces électrodes peuvent être des électrodes jetables ou des électrodes sèches. De préférence, le support est adapté pour recevoir entre une et cinq électrodes ou plus d'électrodes. Le signal ECG est de très faible niveau (mV) et présente une bande de fréquence utile comprise entre 0.05 Hz et 100 Hz.

On va maintenant décrire plus en détail le principe de fonctionnement du dispositif.

Les moyens de contrôle 15 vérifient le niveau énergétique (par exemple piles) des moyens d'acquisition. Les moyens de contrôle peuvent également envoyer un message de test aux différents capteurs pour tester la communication entre les moyens de contrôle et les capteurs.

Les moyens de contrôle commandent l'acquisition simultanée des signaux par les trois capteurs (capteur ECG, capteur acoustique et capteur de pression). L'acquisition simultanée des 3 canaux facilite le repérage chronologique des évènements dans le cycle cardiaque. Par ailleurs, l'acquisition simultanée permet de définir un certain nombre d'intervalles de temps entre des évènements relatifs à différentes ondes enregistrées par les capteurs afin de déterminer différentes phases de la révolution cardiaque.

Le signal provenant du capteur 11, encore dénommé signal d'électrocardiogramme, ou bien signal ou courbe « ECG », correspond à un cycle cardiaque. Il est composé de cinq ondes caractéristiques d'événements cardiaques, à savoir :

- l'onde P qui reflète la dépolarisation des oreillettes induisant leurs contractions simultanées,
- le complexe formé par les ondes Q, R et S, qui est caractéristique de la dépolarisation des ventricules ; et

- l'onde T, qui exprime le phénomène de repolarisation des ventricules.

Afin d'en faciliter l'exploitation, le signal ECG est tout d'abord avantageusement amplifié et filtré. Le complexe QRS peut par exemple être traité, conformément à l'enseignement de FR 2 983 055. Selon un mode de réalisation rapide, basé sur la méthode SFNL (Schéma de Filtrage Non Linéaire) évoquée dans FR 2 983 055, une détection de toutes les ondes peut être opérée en combinaison avec le PCG, selon un mode de fenêtrage.

Par ailleurs, selon un mode de réalisation avantageux de la présente invention, on traite l'onde T au moyen d'une méthode par morphologie mathématique multi échelles, ou « 3M ». Cette méthode, de type connu en soi, est déjà décrite dans P. Maragos, R. W. Schafter, and M. A. Butt,Mathematical Morphology and Its Applications to Image and Signal Processing. Norwell, MA:Kluwer, 1996. Cette méthode permet une analyse quantitative de structures géométriques, permettant d'extraire des informations relatives à la forme et à la taille grâce à des éléments structurants

Comme cela est connu, cette méthode fait intervenir des phases successives de dilatation, d'érosion, d'ouverture et de fermeture. Chacune de ces phases est mise en oeuvre, en utilisant les éléments structurants précités. La forme de chacun de ces éléments, son amplitude et sa longueur affectent la sortie du filtre morphologique. La clé d'extraction de pics, avec une erreur moyenne minimale, est l'utilisation d'un élément de structure parfaite et adaptée.

On connait déjà un procédé de détection du complexe QRS de l'ECG, au moyen d'une telle méthode. Le complexe QRS a une forme très caractéristique et un pic assez différencié permettant de le détecter plus facilement que les autres ondes de l'ECG. Ce procédé est décrit notamment par P. E. Trahanias dans « An approach to QRS detection using mathematical morphology » IEEE Trans Biomed Eng, vol 40, no2, pp 201-205, Feb. 1993. Ce procédé multi-échelle consiste en l'application répétitive des opérateurs morphologiques, en variant la forme et la taille des éléments structurants. Ce procédé de détection du complexe QRS n'est pas applicable de façon simple à la détection de l'onde T de l'ECG, en particulier en ce qui concerne ses éléments structurants.

En effet, le complexe QRS de la courbe ECG a une forme triangulaire. Par conséquent, l'élément structurant est choisi triangulaire avec une amplitude similaire à l'amplitude du pic R. Après avoir fixé l'amplitude, on doit choisir la longueur et la pente d'une manière qui correspond à tous les types de pics R. Les résultats ont montré qu'un élément structurant plus long conduit à une meilleure réduction du bruit, mais une réduction aussi de l'amplitude QRS et des ondes T et U. Quand il s'agit de la pente, les pentes les plus petites permettent plus de réduction de bruit, mais aussi plus la réduction d'amplitude du signal.

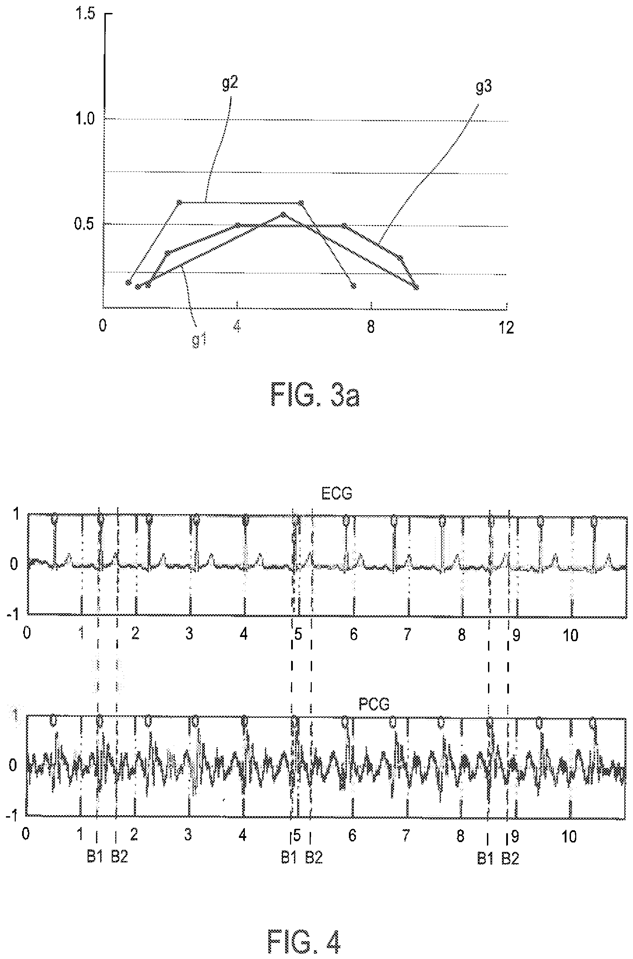

Il est du mérite des inventeurs de la présente invention d'avoir identifié des éléments structurants qui, tout en étant adaptés au traitement de l'onde T, peuvent être mis en oeuvre dans une méthode qui demande relativement peu de cycles de calculs. Ces éléments structurants sont illustrés sur la figure 3a, sur laquelle les longueurs des éléments de structure sont portées en abscisses et les amplitudes de ceux-ci sont portées en ordonnées. On retrouve un premier élément g1 qui est un triangle de petite amplitude et de grande longueur, ainsi que deux autres éléments g2 et g3, en forme d'hexagone. L'élément g2 présente une plus grande amplitude que celui g3, mais aussi une longueur plus faible. Une telle méthode est rapide et précise, tout en étant moins lourde, ce qui la rend adaptée aux appareils mobiles, comme une tablette ou un Smartphone.

L'algorithme est simple et la méthode ne décale pas les ondes recherchées. L'algorithme en blocs est le suivant :

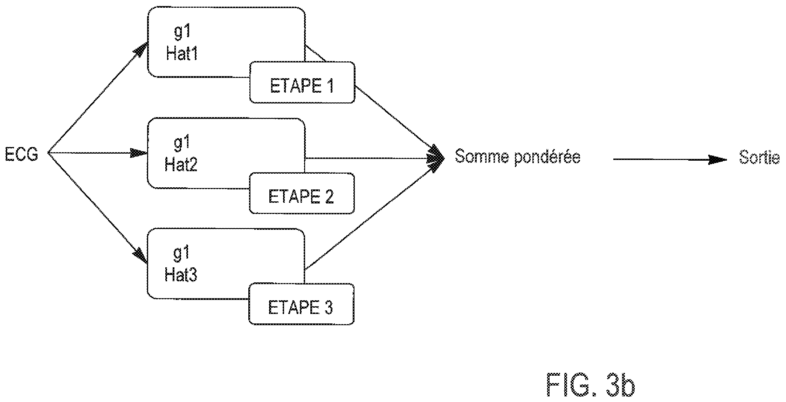

1-Filtrage de type « 3M » selon la figure 3b.
Sur cette figure, Hat(j)= Top hat(j) + Bottom hat(j), pour j variant de 1 à 3
2-Différentiation et accumulation
3-Seuillage et décision

Le début et la fin de l'onde QRS sont trouvés relativement au pic R en appliquant une simple fenêtre fixée depuis la réglette numérique, décrite dans ce qui suit et/ou par la méthode modifiée SFLN. Le même processus est réalisé pour l'onde T.

Le signal provenant du capteur 12, encore dénommé signal phonogramme, ou bien signal ou courbe « PCG », est composé de plusieurs zones, ou bruits caractéristiques d'événements cardiaques, à savoir :

- le premier bruit ou son B1, qui est synchronisé après le pic R de la courbe ECG. Typiquement, sa durée totale est de 100 ms (millisecondes) et sa bande fréquentielle est de 91 Hz à 179 Hz. Il est composé de l'oscillation T1, correspondant à la fermeture des valves tricuspide, ainsi que de l'oscillation M1, correspondant à la fermeture des valves mitrales ;
- le deuxième bruit ou son B2, qui est synchronisé sur la fin de l'onde T de la courbe ECG. Typiquement, sa durée totale est de 50 ms à 100 ms et sa bande fréquentielle est de 145 Hz à 200 Hz. Il est composé de l'oscillation A2, correspondant à la fermeture de la valve aortique, ainsi que de l'oscillation P2, correspondant à la fermeture de la valve pulmonaire. Normalement, les valves aortiques ferment avant les valves pulmonaires, de sorte qu'il y a un temps de retard, dénommé « split », entre ces composantes A2 et P2 ;

- éventuellement un troisième bruit ou son, situé en phase diastole et débutant après OM, ainsi qu'un quatrième bruit, synchronisé sur l'onde P de la courbe ECG.

Un des aspects de la présente invention est la localisation automatique des deux bruits B1 et B2, à savoir sans intervention d'un quelconque opérateur. On doit en effet garder à l'esprit que le signal brut, non traité, n'est pas exploitable en l'état. On se référera à cet égard à la figure 4, qui illustre les courbes ECG et PCG non traitées.

En vue de cette localisation automatique, on décompose tout d'abord la courbe PCG selon la méthode connue en soi, dite de Décomposition Modale Empirique (ou "EMD", sigle de l'expression anglo-saxonne "Empirical Mode Decomposition"). Cette étape conduit à la formation d'une succession de courbes décomposées, de niveau de décomposition incrémental, qui sont dénommées Fonctions Modes Intrinsèques (ou "IMF", sigle de l'expression anglo-saxonne "Intrinsic Mode Functions"). Ces IMF sont référencées IMF1 à IMFi, où i est en général compris entre 20 et 100, selon le niveau de décomposition.

Conformément à l'invention on sélectionne la ou les IMF qui, en pratique, sont les plus intéressantes en termes d'informations fournies. A cet effet, on applique le critère de sélection modale Rj, tel que défini ci-dessous :

$$R_j = 1 - \left[ E(IMF^2{}_j) / E(s^2(k)) \right]$$

$E(IMF^2{}_j)$ désigne l'énergie de chaque IMF, pour j variant de 1 à n, où n est le nombre d'IMF obtenues lors d'une décomposition EMD donnée. $E(s^2(k))$ désigne l'énergie globale du signal initial, à savoir non décomposé. Les énergies de signal sont calculées de façon connue en soi.

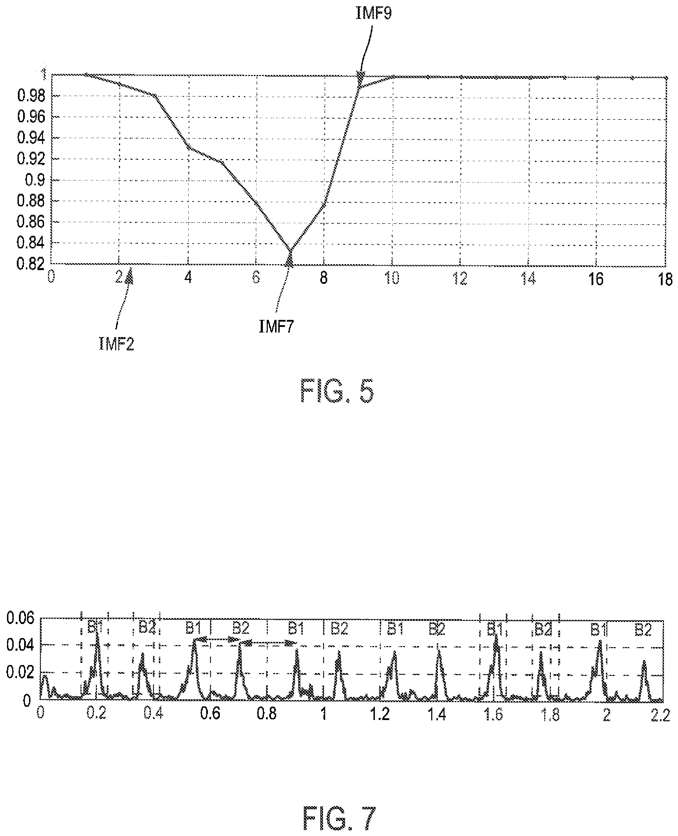

La figure 5 illustre la variation de la valeur du critère Rj avec le quantième des IMF obtenues. Comme le montre cette figure, les IMF 2 à 9 présentent une énergie de signal significative, avec un maximum pour l'IMF7, alors que les autres IMF possèdent une énergie sensiblement nulle. On retient donc, pour la suite du traitement conforme à l'invention, les IMF précitées allant de IMF2 à IMF9.

A titre de variante, on peut retenir les IMF pour lesquelles la valeur du critère Rj est inférieure à un seuil prédéterminé, bien inférieur à 1, en tenant compte des contraintes fréquentielles liées à la bande fréquentielle de B1 et de B2, de manière à retenir un nombre plus faible d'IMF préférées. Dans ce cas, on pourra retenir uniquement les IMF comprises entre l'IMF4 et l'IMF7 de la figure 5.

Pour l'analyse des signaux, conformément à l'invention on sélectionne la ou les IMF qui, en pratique, sont les plus intéressantes en termes d'informations fournies. En variante, ce seuil peut donc être de type dynamique, à savoir par exemple Rj(seuil)=(min Rj + 1)/2 . On conserve uniquement les IMF dont le Rj est inferieur à ce seuil afin de retenir les IMF intéressantes pour l'objectif recherché.

Pour la détermination précise des paramètres sur un type de signal, on renforce le critère de sélection Rj, en introduisant des critères supplémentaires basés sur des informations connues a priori sur le type des signaux, notamment leur contenu fréquentiel et/ou leur durée et/ou leur position dans le temps. A titre d'exemple, dans la recherche de la position de B1 et de B2, on n'a retenu que les IMF dont le contenu fréquentiel se situe dans la bande comprise entre 20 et 200 Hz.

Le seuil Rj de sélection des IMF peut être fixe, par exemple égal à 90%. Ce seuil est adapté à une étape préliminaire de debruitage du signal. En effet, L'EMD peut être utilisée du fait de sa décomposition multi échelles pour le débruitage des signaux. Après décomposition du signal par l'EMD, toutes les IMF dont le Rj est inférieur à 90% sont alors conservées dans l'étape de reconstruction du signal.

Cette sélection des IMF préférées va être utilisée pour détecter automatiquement la position des bruits B1 et B2. Cette détection peut être mise en œuvre de deux manières, conformément à l'invention. Selon une première variante, elle fait intervenir une synchronisation avec la courbe ECG. Selon une variante alternative, cette détection est réalisée en utilisant la THH (ou Transformée de Huang Hilbert), indépendamment de la courbe ECG.

Selon la première variante, il s'agit tout d'abord de détecter les zones caractéristiques de l'ECG par toute approche appropriée. De façon avantageuse, pour le complexe QRS de l'ECG, une telle approche est par exemple de type WTMM combiné à la méthode SFNL (Schéma de Filtrage Non Linéaire), telle que décrite dans FR 2 983 505, méthode très précise même dans le cas d'ECG bruités.

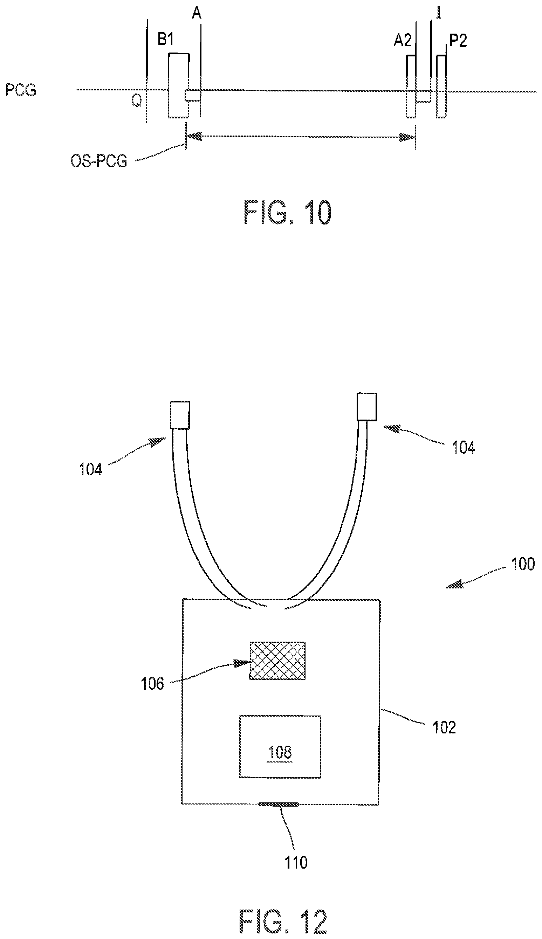

Pour détecter l'onde T, on utilisera avantageusement la méthode, de type morphologie mathématique multi-échelles, décrite ci-dessus. Pour détecter l'onde P (et aussi les autres ondes de l'ECG) la méthode SFNL a été modifiée en l'appliquant à tout le signal puis seuillée pour trouver les pics R. Cette méthode SFNL est ensuite utilisé dans une fenêtre délimitée relativement aux pics R et en utilisant les positions sur la réglette numérique de la figure 10, afin de détecter précisément l'onde P, maximum, début et fin.

Puis on extrait les IMF les plus intéressantes, selon le procédé décrit ci-dessus, par utilisation du critère Rj. De façon avantageuse, chaque IMF d'intérêt est soumise à un filtrage passe-bande, de façon connue en soi. On garde ainsi uniquement les IMF, qui appartiennent à une plage de fréquences prédéterminée. A titre d'exemple, pour localiser les bruits B1 et B2, cette plage est par exemple comprise entre 20 et 200 Hz. Si d'autres critères de localisation doivent être appliqués, la valeur de cette plage pourra être modifiée.

Puis on reconstitue un PGC, dit utile, à partir des IMF d'intérêt, appartenant à la plage de fréquence éventuellement choisie. Ce PCG utile est par exemple égal à la somme des IMF d'intérêt (par exemple IMF2 à IMF9), selon la formule suivante :

$$\sum_{j=2 \text{ à } 9} (IMFj)$$

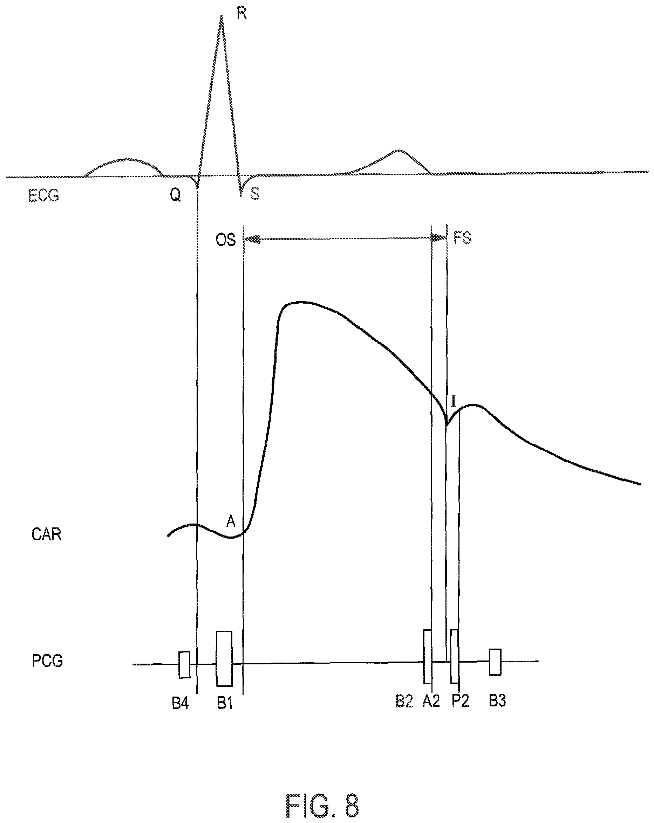

On synchronise ensuite l'ECG et le PCG, qui ont été traités selon la procédure ci-dessus, ce qui permet de localiser les bruits B1 et B2. Le début du premier bruit B1 correspond à la fin du pic R de la courbe ECG, alors que la fin de ce premier bruit permettra la localisation du pied de l'onde CAR (voir plus loin en référence à la figure 8). Le début du deuxième bruit B2 correspond à la fin du pic T de la courbe ECG, alors que la fin de ce deuxième bruit permettra la localisation de l'incisure catacrote sur la courbe CAR.

Les bruits B1 et B2, localisés comme expliqué ci-dessus, sont ensuite analysés, selon toute procédure appropriée, connue en soi. Par exemple, une analyse fréquentielle permet de visualiser leurs oscillations et de détecter leurs extrema. Une analyse à base de spectrogramme ou avec plus de précision un scalogramme (transformée en ondelettes) permet de détecter la distance temporelle entre les pics des bruits de fermeture aortique et pulmonaire (A2 et P2)

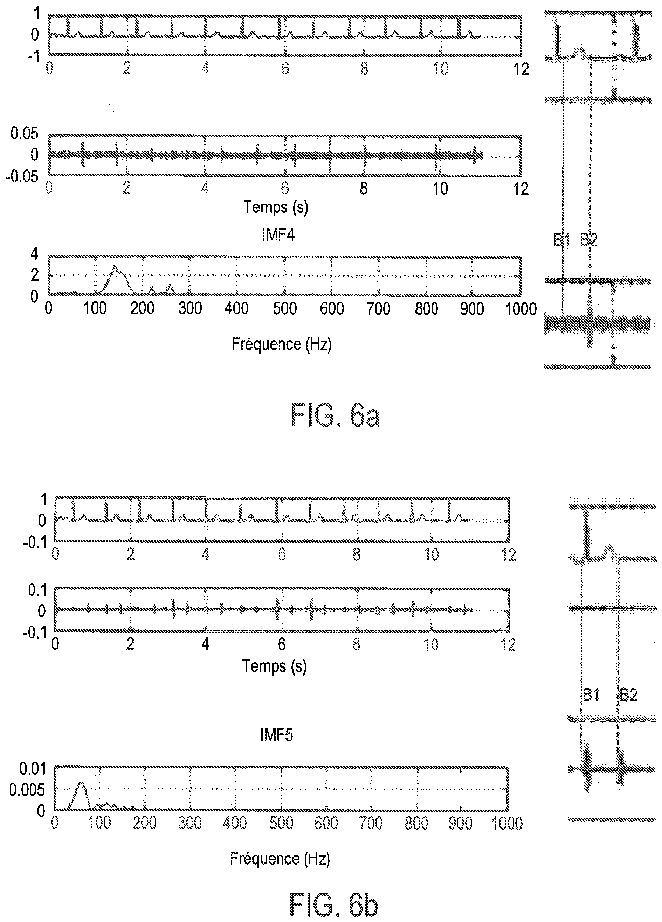

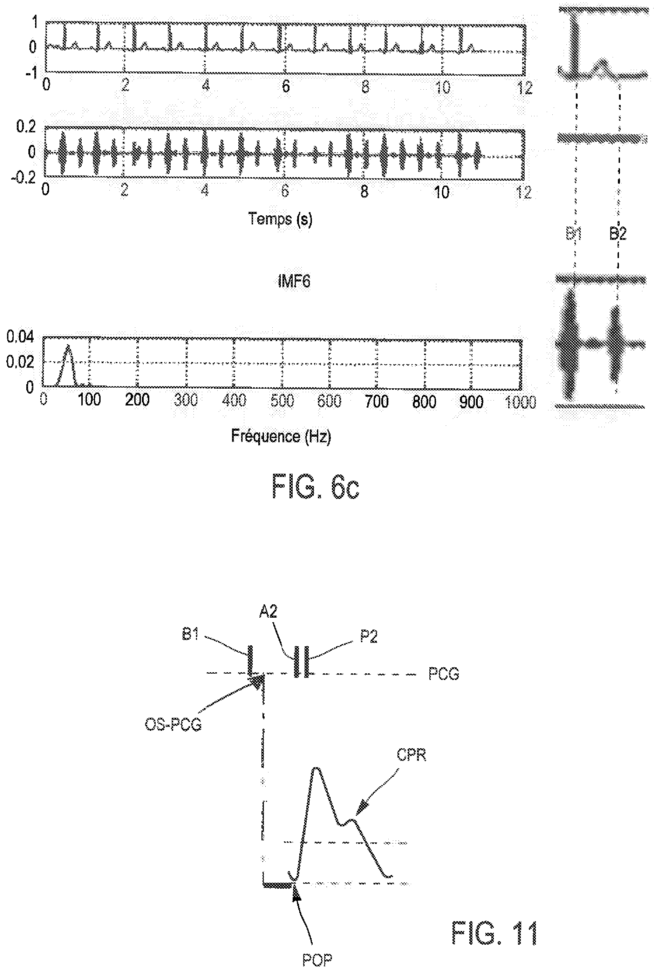

Sur les figures 6a à 6c, on a représenté l'usage du critère fréquentiel conjugué à celui du critère Rj pour la détection précise des positions B1 et B2. A titre de variante, on peut choisir un PCG utile, tel que défini ci-dessus, qui est égal à la somme des IMF les plus intéressantes en termes d'énergie et d'informations fournies.

La deuxième variante diffère essentiellement de la première variante ci-dessus, en ce qu'elle ne fait pas intervenir d'acquisition d'une courbe ECG. Selon cette variante, on extrait tout d'abord les IMF les plus intéressantes, par utilisation du critère Rj, on garde optionnellement les IMF qui appartiennent à une plage de fréquences déterminée, puis on reconstitue un PGC utile. Ces étapes sont mises en oeuvre comme décrit ci-dessus, pour la première variante.

Ensuite on applique la transformée de Huang Hilbert sur le PCG utile, ce qui permet d'obtenir l'enveloppe modale de ce PCG utile. On détecte ensuite lesmaxima de l'enveloppe modale ainsi obtenue, puis on réalise une opération de seuillage, de type connu en soi. Ceci permet de localiser les bruits B1 et B2.

On réalise ensuite des opérations de fenêtrage et d'analyse des bruits B1 et B2, localisés comme expliqué ci-dessus. Ceci permet de localiser les maxima des oscillations et de mesurer les écarts temporels et d'amplitudes (voir figure 7).

L'intérêt de cette deuxième variante est double :

- Les sons B1 et B2 peuvent être localisés indépendamment de la courbe ECG.
- Des souffles systoliques et diastoliques peuvent être détectés, grâce aux enveloppes entre les sons B1 et B2.
- Le rythme cardiaque peut être calculé.

Le signal provenant du capteur 13, qui est représentatif de la pression artérielle carotidienne, est aussi dénommé signal ou courbe « CAR » (à savoir carotidien). Il comprend successivement:

- un minimum absolu, qui correspond à la pression sanguine diastolique
- une portion croissante qui correspond à la contraction du coeur
- un maximum absolu, qui correspond à pression sanguine systolique
- une portion décroissante qui se termine en un minimum relatif, ou incisure catacrote, qui correspond à la fermeture de la valve aortique.

La pression sanguine retourne à la valeur minimale absolue initiale, puis le cycle recommence.

Conformément à l'invention, après filtrage et débruitage des courbes ECG et PCG, on met en correspondance les trois courbes ECG, PCG et CAR acquises selon le procédé décrit ci-dessus (voir figure 8). Ce filtrage et ce débruitage peuvent être mis en oeuvre par l'un ou l'autre des traitements décrits ci-dessus, ou encore par tout autre traitement mathématique approprié.

Puis, on réalise tout d'abord un calage des courbes ECG et PCG. La localisation du pic de l'onde R correspond au premier bruit B1, alors que la localisation du pic de l'onde T correspond au deuxième bruit B2. On détermine ensuite plusieurs paramètres représentatifs de l'activité. A cet effet, on mesure certaines durées caractéristiques, en utilisant à la fois les différentes courbes ECG, CAR et PCG.

La durée A2-I a pour début le point A2 de la courbe PCG, soit la fermeture de la valve sigmoïde de l'aorte. Le point I de la courbe CAR, soit la fin de cette durée, traduit le même phénomène, à savoir qu'il correspond à l'arrivée de l'onde générée par cette fermeture. En d'autres termes, cette durée A2-I correspond au temps de transit de l'onde pulsatile, depuis l'orifice aortique où elle prend sa naissance, jusqu'au point de mesure sur la carotide.

Cette durée A2-I permet d'accéder à la vitesse d'onde de pouls VOP, par le calcul suivant :

VOP = D / A2-I, où D est la longueur du segment artériel reliant le capteur 12, positionné sur l'aorte, et le capteur 13, positionné sur le cou. En pratique, cette longueur est mesurée directement sur le patient.

Le temps TE, ou temps d'éjection, sépare l'ouverture sigmoïde OS et la fermeture sigmoïde FS, auxquelles on a accès à partir du signal CAR.

Le temps QB1, ou temps de transformation, sépare le minimum de l'onde Q de l'ECG et le début du bruit B1 du PCG.

Le temps QB2, ou durée de systole électromécanique, sépare le minimum de l'onde Q de l'ECG et le début de la composante A2 du bruit B2 du PCG.

Le temps PPE, ou période de pré éjection, est la différence entre QB2 et TE.

Le temps CI, ou contraction isovolumique, est la différence entre PPE et QB1.

Le coefficient hémodynamique CH est égal au rapport (TE/PPE) entre le temps d'éjection TE et la période de pré éjection PPE.

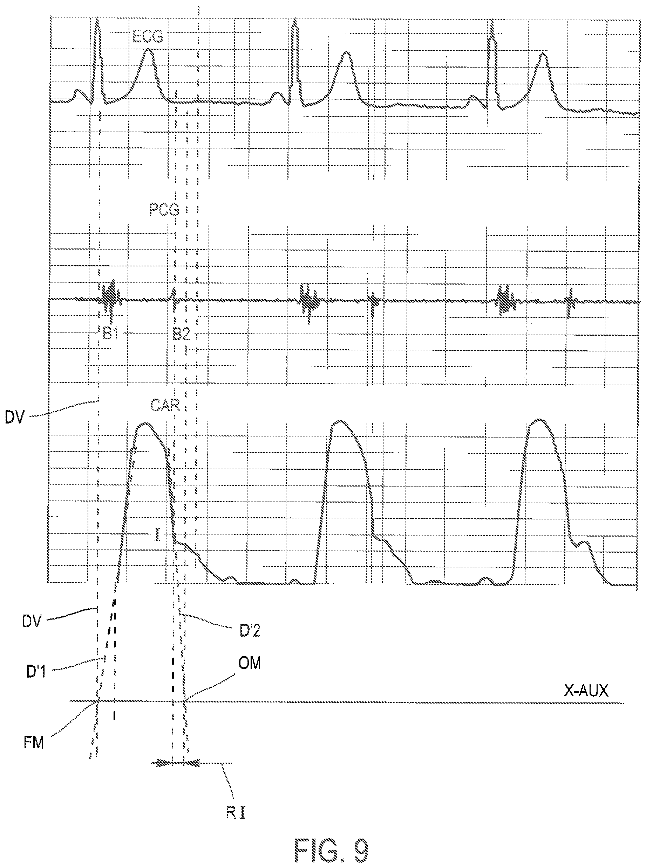

La durée RI de relaxation isovolumétrique est calculée en référence à la figure 9, laquelle montre les courbes ECG, PCG et CAR disposées les unes sur les autres. On réalise tout d'abord une droite D'1 d'interpolation d'une première partie de la courbe de pression, en l'occurrence de la partie croissante de cette courbe. Cette interpolation peut être de tout type approprié, linéaire ou analogue. On trace par ailleurs une droite verticale DV, à savoir perpendiculaire à l'axe des temps, qui passe par le bruit B1 de la courbe PCG. L'intersection entre cette droite verticale et la droite d'interpolation correspond au point de fermeture mitrale FM.

On détermine ensuite la pente de cette droite d'interpolation, puis on réalise une deuxième droite D'2 d'interpolation d'une autre partie de la courbe de pression, en l'occurrence de la partie décroissante de cette courbe. Les pentes de ces deux droites D'1 et D'2 sont avantageusement égales. Par ailleurs, on trace une droite horizontale dite auxiliaire X-AUX, à savoir parallèle à l'axe des temps, qui passe par le point FM. L'intersection entre cette droite horizontale et la deuxième droite d'extrapolation correspond au point d'ouverture mitrale OM. Ceci permet d'accéder à la durée de relaxation iso volumétrique, correspondant au temps entre l'incisure catacrote de la courbe de pression et le point de fermeture mitrale.

La figure 10 illustre une variante avantageuse de l'invention, selon laquelle on place le point correspondant à l'ouverture de la valve sigmoïdienne sur la courbe PCG. Cette ouverture est matérialisée par le point A sur la courbe CAR de la figure 8. On connait par ailleurs le temps de transit A2-I, comme vu ci-dessus. L'ouverture sigmoïdienne, au niveau du coeur, se produit donc à un temps correspondant à ce point A, dont est retranchée la durée A2-I. Ce point OS-PCG, qui est identifié sur la courbe PCG elle-même, constitue donc un repère initial matérialisant l'ouverture de la valve sigmoïdienne. Il peut servir de base de temps pour déterminer toutes les durées caractéristiques listées ci-dessus. Sur cette figure 10, on retrouve donc une sorte de « réglette numérique », qui regroupe un ensemble de points caractéristiques de la chronologie du cycle cardiaque, permettant le calcul de paramètres cardiaques et/ou vasculaires.

Dans ce qui précède, on a calculé la vitesse d'onde de pouls en plaçant le capteur de pression au niveau de la carotide. L'utilisation du temps de transit A2-I, faisant intervenir l'incisure catacrote, est bien adaptée dans ce cas puisque cette incisure est parfaitement visible sur la courbe de pression CAR.

Si on désire calculer la vitesse d'onde de pouls au niveau d'autres sites du corps humain, et que l'incisure catacrote n'est pas visible sur la courbe de pression, on prendra judicieusement le repère initial OS-PCG de l'ouverture sigmoïdienne, ou pied de l'onde, sur la réglette numérique de la figure 10. OS-PCG peut être distinct du bruit B1, comme illustré sur cette figure 11. En haut de la figure 11 on a représenté, de façon schématique et à plus petite échelle, la réglette numérique de la figure 10. On a placé, sur cette figure 11, les bruits B1, A2 et P2. On mesure alors la durée entre ce repère initial OS-PCG et le pied POP de l'onde de pouls de la courbe de pression CPR, afin d'accéder à la VOP. Il y a, là encore, mesure d'un temps de transit qui s'effectue d'une façon différente de la figure 8.

Selon une autre variante non représentée de l'invention, les deux sites de mesures peuvent ne pas inclure le cœur. Dans ce cas la mesure peut être faite en différé en déplaçant le capteur de pression du premier site au second site, avec recalage sur la réglette numérique de la figure 10.

Les données obtenues en sortie des moyens de traitement peuvent être transmises sur un serveur distant, par exemple en utilisant un protocole Internet sécurisé. Ceci permet au praticien de consulter des courbes correspondant aux différentes ondes acquises par les moyens d'acquisition, ainsi que de consulter les paramètres calculés par les moyens de traitement afin de faciliter l'établissement d'un diagnostic. Avantageusement, ces courbes peuvent être comparées à un modèle prédéterminé ou comparées à des courbes issues d'acquisitions précédentes sur le patient. Ceci permet au praticien de vérifier l'évolution cardiaque du patient.

Un mode de réalisation tout particulièrement avantageux du procédé de l'invention, va maintenant être décrit. Dans le but de trouver des intervalles courts, soit un temps de transit typiquement inférieur à 30 ms, avec au moins un capteur acoustique et un capteur de pression en mesure simultanée, il faut lever la « subjectivité » du stéthoscope (usage d'une onde acoustique cardiaque numérique, donc stéthoscope électronique, avec moyens de traitements numériques).

Dans ce qui suit, l'abréviation «PH » correspond à un phénomène physiologique particulier. De plus, les valves dont il est fait mention se trouvent toutes sur le site cœur.

Etape 1 : Sur la courbe CAR (site cou) : déterminer l'incisure catacrote : point I (PH : fermeture des valves aortiques décalée)

Sur la courbe PCG (site cœur) : déterminer le point A2 (correspondant au même phénomène : fermeture des valves aortiques, non décalée).

Etape 2 : Le temps de transit de l'onde de pouls qui se propage du cœur au site de mesure de CAR est l'intervalle de temps A2-I (autre notation tc)

Etape 3 : Déterminer le pied de l'onde sur CAR : point OA' (PH : ouverture des valves aortiques décalée)

Etape 4 : Sur le PCG, recalage du pied de l'onde OA' par décalage vers la gauche égal au temps de transit tc (voir Etape 2), donc point OA (PH : ouverture des valves aortiques non décalée).

Etape 5 : Le recalage des points caractéristiques liés à des évènements physiologiques cardiaques sur le même axe temporel permet d'avoir une empreinte cardio-vasculaire de l'individu, ou réglette numérique cardiaque. Celle-ci permet de déterminer des intervalles précis relatifs à l'état cardiaque et/ou à un état artériel et d'être prise par ailleurs comme origine des temps pour la propagation de l'onde de pouls : instant t0 lié à la naissance de l'onde de pouls (points Q ou OA ou FA, selon la méthode de calcul en usage pour le calcul du temps de propagation de l'onde de pouls).

Etape 6 : Le temps de transit permet de calculer une VOPc =D/tc, où D est la distance entre les deux sites de mesure.

Etape 7 : Le sélecteur modal introduit dans le calcul de l'EMD permet le débruitage du signal PCG sans délocalisation. Le sélecteur modal permet de sélectionner les échelles utiles de la décomposition en les croisant avec une connaissance a priori sur le contenu fréquentiel de l'évènement recherché.

Etape 8 : L'application de l'EMD (selon l'étape 6) avec le calcul de l'enveloppe et les contraintes fréquentielles des bruits B1, respectivement B2, permet de délimiter les intervalles des bruits B1 et B2 et d'indiquer la présence de souffles systoliques ou diastoliques même sur le PCG seul.

Etape 9 : En utilisant en outre l'ECG comme référence temporelle pour le PCG, la localisation des intervalles B1 et B2 est précisée : après l'onde R pour le B1, après l'onde T pour le B2. L'onde R et l'onde T de l'ECG sont estimées rapidement grâce à l'enseignement de la méthode 3M. Les ondes B1, respectivement B2, comprennent plusieurs oscillations qu'il est difficile de distinguer.

Etape 10 : la transformée en ondelettes permet dans l'intervalle délimité par l'étape 7, précisé par l'étape 8, de distinguer la composante mitrale de la composante tricuspide dans l'intervalle B1, respectivement la composante aortique de la composante pulmonaire dans l'intervalle B2.

Etape 11 : La connaissance acquise à l'étape 9 permet de préciser les points recherchés : M1 (ou FM, fermeture de la valve mitrale) et A2 (ou FA, fermeture de la valve aortique).

Etape 12 : La connaissance du pied de l'onde, de l'incisure catacrote sur la courbe CAR et de M1 (ou FM selon l'étape 11) permet de calculer par interpolation le point OM correspondant à l'ouverture des valves mitrales.

Etape 13 : Le calcul du paramètre CI, indépendant de la fréquence, caractéristique de la contractilité cardiaque, est obtenu par l'intervalle compris entre les points FM et OA.

Etape 14 : Le calcul de la relaxation iso volumétrique RI est calculé par l'intervalle entre les points FA et OM.

Etape 15 : D'autres paramètres cardiaques peuvent être calculés, en plus de ceux déjà obtenus :

$$\text{Index systolique} = \text{PEP/TE}$$

$$\text{MPI} = (\text{RI+CI}) / \text{TE}$$

Temps de Diastole = intervalle (OM, FM du cycle suivant),

Temps de remplissage= Temps de diastole - RI

Temps de remplissage accéléré = intervalle (début de l'onde P et FM du cycle suivant)

$$\text{Index diastolique} = \text{RI/ Temps de remplissage.}$$

De même la précision de la présence des bruits pathologiques B3 et B4

Ainsi, pour calculer une VOP sur un autre site de mesure que la carotide, par exemple fémoral, il est possible d'utiliser les mêmes méthodes que la littérature en prenant un autre capteur de pression ou en se recalant à l'ECG. Dans ce contexte de méthodes, il est avantageux de se recaler sur la réglette numérique qui procure des points initiaux systoliques et diastoliques. La présente invention propose une nouvelle approche : connaissant le temps tc (étape 2) et le CI qui est indépendant de la fréquence (étape 13), dans une première mesure simultanée, on déplace dans un deuxième temps le capteur de pression sur le site fémoral soit en simultané : PCG sur le cœur, calcul de M1, décalage de CI et on obtient le pied de l'onde OA sur le PCG. Avec le capteur de pression sur le site fémoral, on détermine le pied de l'onde décalée OA".

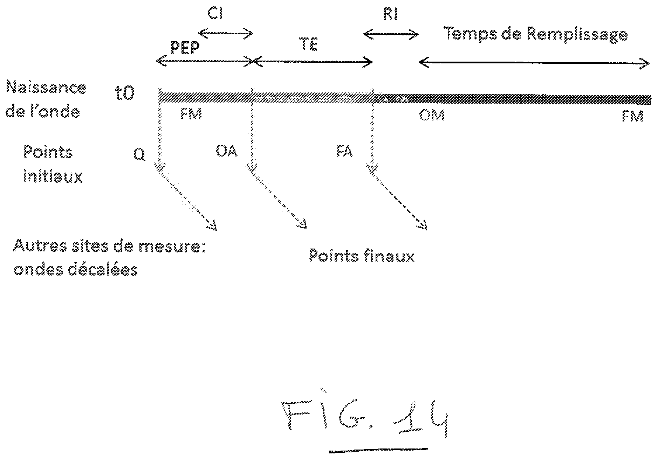

La VOP est égale à Df / tf, où tf est l'intervalle de temps entre OA et OA", et Df la distance entre les sites de mesure. Cette méthode est plus cohérente avec la distance parcourue par l'onde, le sens de propagation de l'onde et le temps mis pour faire ce parcours, aspects non résolus dans l'état de la technique. Ainsi on peut accéder à d'autres paramètres hémodynamiques déjà connus dans la littérature, tels que l'index d'amplification, ainsi qu'à des variantes conformes aux attentes des médecins sur la base de ces enseignements. Sur la figure 14, on retrouve une schématisation des fonctionnalités de la réglette numérique ci-dessus.

Le mode de réalisation décrit ci-dessus correspond à une mise en oeuvre tout particulièrement avantageuse de l'invention. Néanmoins, le procédé conforme à l'invention trouve également son application à certaines étapes individuelles, parmi celles 1 à 15 décrites ci-dessus. L'invention a donc, en d'autres termes, également pour objet toute combinaison techniquement compatible entre certaines de ces étapes individuelles.

A titre de variante avantageuse de l'invention, représentée à la figure 12, on peut utiliser une lyre pérfectionnée. Une lyre traditionnelle, symbole par excellence du médecin, a une ergonomie qui a été étudiée de longue date, adaptée à l'exercice et au confort du médecin : souplesse, stabilité par rapport à son placement au niveau de l'oreille et autour du cou en cas de non-utilisation.

On connait, par le brevet américain 7 346 174, une lyre pourvue d'un microphone, laquelle est reliée à un stéthoscope équipé d'un moyen d'enregistrement numérique de la dictée. Contrairement à cet art antérieur, la lyre conforme à l'invention est avantageusement autonome par rapport au stéthoscope.

De façon plus précise, la lyre 100 de la figure 12 comprend un corps 102 et deux écouteurs 104. Le corps 102 est équipé d'un microphone 106, de moyens de traitement électroniques 108, ainsi que d'un port 110 de type USB (Universal Serial Bus). Les moyens de traitement comprennent des moyens d'enregistrement de la dictée reçue par le microphone 106, des moyens de filtrage du son, ainsi que des moyens de connexion au ou à chaque capteur acoustique.

De façon préférée, ces moyens de connexion sont de type « sans fil » et font par exemple intervenir une connexion « Bluetooth ». De la sorte, la lyre est d'une légèreté satisfaisante et peut être portée autour du cou, de façon confortable. Ce mode de communication lui permet de s'interfacer ou communiquer avec tout autre support numérique externe intégrant le Bluetooth (PC, Tablette ou Smartphone). Le port USB 110 permet de diriger l'enregistrement vers une clé Bluetooth, vers un dictaphone en usage déjà au cabinet médical, ou encore vers tout appareil analogue.

Un mode de fixation «magnétique» sur la lyre sert avantageusement à fixer le capteur de pression 12, et/ou à « supporter » ou « fixer » un microphone externe optionnel pour l'enregistrement des données de type audio. La lyre peut être utilisée pour l'écoute sélective des données d'auscultation provenant du ou de chaque capteur acoustique 11. Grâce aux moyens de filtrage précités, la lyre procure avantageusement à l'utilisateur une sélection de l'écoute basse fréquence, moyenne fréquence ou haute fréquence adaptée à l'écoute et à la mesure ou à un message audio. En particulier, il peut sélectionner l'écoute des sons pulmonaires seuls, lors d'une auscultation pulmonaire, ou bien des sons cardiaques seuls en auscultation cardiaque. Le praticien peut également régler le niveau d'écoute, grâce à une fonction d'amplification.

Conformément aux recommandations actuelles, en particulier lorsqu'un acte médical est partagé, comme c'est le cas en télémédecine, les détails de l'auscultation et les éventuels incidents au cours de l'auscultation doivent être notés avec cette auscultation, y compris la date et l'heure. Cette auscultation se visualise/s'analyse en temps réel, de même les commentaires oraux peuvent être avantageusement émis en temps réel en parallèle avec le déroulement de l'auscultation. Dans le cas de l'invention, cette dernière est de type tri modal, à savoir qu'elle est plus complexe que l'auscultation par stéthoscope uniquement.

La lyre conforme à l'invention permet de faire ces commentaires simultanément à l'auscultation et de les attacher au dossier numérique d'auscultation, qui est lui-même enregistré sous le dossier personnel du patient, en temps réel. Cela permet, en outre, au médecin de rester sur une auscultation moyenne de 15 à 20 minutes. Cela permet au médecin d'envoyer en quasi instantané, d'un seul geste, les données de l'auscultation, les commentaires, heure, date et les autres données qui font partie de la consultation en cours (poids, sexe, âge, médicaments...).

La lyre conforme à l'invention permet d'écouter les enregistrements antécédents, mémorisés dans sa mémoire SD ou à partir de la base de données du logiciel. Cette lyre permet aussi d'enregistrer un rapport, puis de le lire en différé.

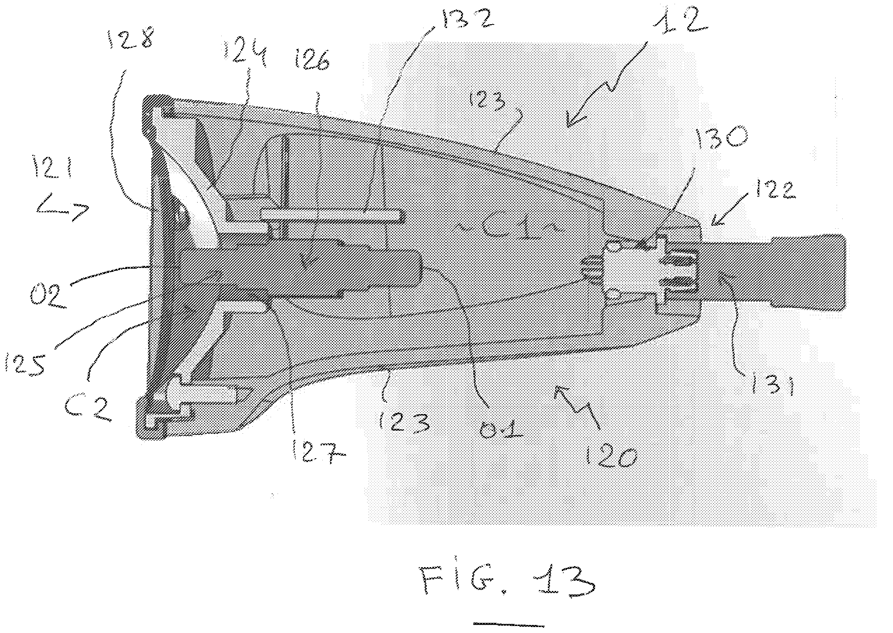

La figure 13 illustre une variante de réalisation avantageuse du capteur de pression 12, représentée de façon sommaire sur la figure 1.

Ce capteur comprend un corps triangulaire 120, dont on note 121 la base, 122 la pointe et 123 les parois latérales. La base 121 supporte un fond concave 124, rapporté de manière étanche par tout moyen approprié. Ce fond 124 est creusé d'une ouverture centrale 125, dans laquelle est reçu un transducteur 126, de tout type approprié. Ce transducteur est maintenu, de manière étanche, contre les parois de l'ouverture 126 par un élément d'étanchéité 127.

Le fond 124 délimite donc deux chambres C1 et C2, dont l'une correspond au volume intérieur du corps 120, et dont l'autre est mise en communication avec la peau du patient. A cet effet une membrane 128, destinée à entrer en contact avec la peau, est tendue sur ce fond. A titre de variante, on peut prévoir de ne pas utiliser de membrane, de sorte que la chambre C2 est fermée par la peau elle-même. Le transducteur 126 est équipé de deux ports ou orifices opposés, dont l'un O1 débouche dans la chambre C1 et l'autre O2 débouche dans la chambre C2.

La pointe 122 est creusée d'un orifice 130, dans lequel est reçu à étanchéité un moyen de connexion 131, de tout type approprié. Ce moyen de connexion 131 est tout d'abord relié au transducteur 126 par une carte électronique 132. Par ailleurs, ce moyen 131 est relié au moyen de contrôle 15 par tout moyen de communication, que ce soit filaire, « Bluetooth » ou analogue.

Les avantages de ce capteur 12 sont les suivants sont les suivants :

- il peut mesurer les pressions relatives, le vide ou les pressions différentielles entre la pression dans la chambre de mesure et la pression de référence de la chambre C2.
- ce capteur est adaptable à différents types de têtes ou chambres de mesures, permettant la mesure de déplacement mécanique sur plusieurs foyers du corps (têtes larges pour les grosses artères et tête plus réduite pour la mesure de l'apex)
- il permet une mesure très fine de la variation de pression entre 0 et 3,45 kPa (0,5 psi)
- il possède une bonne linéarité
- il présente une efficacité validée sur une plage de fréquence du continu à 2 KHz.

Pour mesurer le déplacement mécanique, des capteurs piézoélectriques ont été utilisés dans l'art antérieur. Ces derniers ne sont pas calibrés et leurs résultats ne sont pas reproductibles de manière satisfaisante. L'impédance de la peau varie pour chaque individu en fonction, essentiellement, des paramètres suivants : la température de la peau, la surface et la pression de contact, la tension (force) de contact, l'humidité de la peau. Aussi, pour avoir une meilleure reproductibilité des mesures et une précision plus fine, le capteur 12 conforme à l'invention est un capteur différentiel de pression pour l'acquisition d'une onde relative au déplacement mécanique de la peau. La référence de pression est celle atmosphérique, au niveau de l'orifice O1.

Le système est à base d'une technologie au silicium micro-usiné intégrant la détection, la compensation de température et d'étalonnage. La stabilité et la précision sont acquises grâce à des techniques de compensation éliminant les dérives dues aux variations thermiques. La plage de pression est comprise entre 0 et 3,45 kPa (0,5 psi). L'écart maximal sur la reproductibilité des mesures est de 0,15% de la pleine échelle. Une sensibilité de 70 mV/psi avec un temps de réponse inférieur à 1 ms et il a été validé dans une plage allant du continu à 2 KHz.

Le capteur est compensé en température, les résultats obtenus sont indépendants de la position sur le corps. Le capteur permet la mesure de pressions dont la variation est faible, typiquement quelques Hz. La chambre C2 a été conçue pour une utilisation optimale. L'amplitude, la morphologie, l'échelle temporelle et/ ou fréquentielle de l'onde de pression sont exploitables. La facilité avec laquelle le pied de l'onde est lisible dépend de la réponse en fréquence du transducteur ainsi que de la qualité du signal.

Le capteur de pression 12 permet l'acquisition d'une onde de pression cardio-vasculaire.

Il est destiné à être positionné au niveau de la carotide, l'apex ou tout autre endroit indiqué sur le corps du patient. Les signaux recueillis par le capteur de pression 12 sont aussi des vibrations qui découlent de l'activité cardiaque.

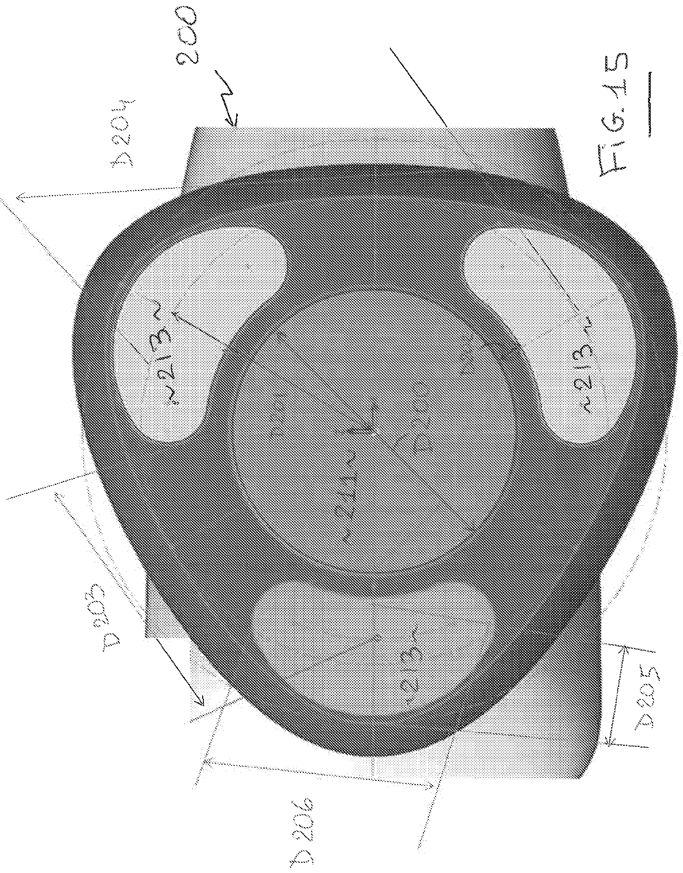

La figure 15 illustre un module 200, dit « module thorax », destiné à être positionné sur le thorax d'un patient. Ce module 200 est composé tout d'abord d'un capteur central 211, destiné à mesurer le PCG. Il est entouré de trois électrodes sèches 213, adaptées pour mesurer l'ECG de façon satisfaisante. Il est prévu une dérivation bipolaire comme base de temps pour le PCG et pour calculer les paramètres cardiaques électriques, ainsi qu'une dérivation bipolaire de confirmation des résultats. Ces deux dérivations sont prises par rapport au neutre, formée par la troisième électrode.

Les distances ont été optimisées pour une mesure la plus satisfaisante possible. On retrouve, sur la figure 15, les distances les plus caractéristiques, dont les valeurs numériques sont données ci-dessous. On notera que ces valeurs sont données à 1% près, une variation plus importante ne permettant plus de donner des résultats satisfaisants :

D200 (diamètre du capteur 211) : 45 mm
D201 (distance entre les centres du capteur 211 et de chaque électrode 213) : 65 mm
D202 (distance entre les bords adjacents du capteur 211 et de chaque électrode 213) : 3 mm
D203 : 29.91 mm
D204 : 72 mm
D205 : 14 mm
D206 : 30 mm.

D'un seul geste, similaire à celui du stéthoscope, le médecin relève un PCG et un ECG synchrones. L'usage du module thorax ci-dessus, simultanément avec le capteur de pression, permet d'acquérir les 3 courbes synchrones :

ECG/PCG/CPR.

**Revendications**

1. Procédé de détermination d'au moins un paramètre représentatif d'une activité cardiovasculaire, comprenant les étapes suivantes :

   - acquisition d'une courbe représentative d'une onde acoustique cardiaque, ou courbe acoustique (PCG),
   - acquisition d'une courbe de type mécanogramme, représentative d'une onde de pression sanguine, ou courbe de pression (CAR), par un capteur de pression (12) adapté pour mesurer des vibrations d'une fréquence inférieure à 5 Hz
   - acquisition d'une courbe représentative d'une onde électrique cardiaque, ou courbe électrique (ECG),
   - mise en correspondance de la courbe acoustique, de la courbe de pression et de la courbe électrique pour estimer au moins un paramètre représentatif d'une activité cardiovasculaire,
   - affichage du ou des paramètre(s) déterminé(s),
   au moins un paramètre représentatif étant une durée représentative d'une activité cardiovasculaire,
   procédé dans lequel on mesure cette durée en prenant, en tant que début de cette durée, l'un (A2) parmi un point de la courbe acoustique (PCG) et un point de la courbe de pression et, en tant que fin de cette durée, l'autre (I) parmi le point de la courbe acoustique et le point de la courbe de pression (CAR), ladite durée étant calculée à partir de deux points mesurés sur deux courbes de morphologies différentes, à savoir la courbe acoustique (PCG) et la courbe de pression (CAR),
   cette mesure permettant de déterminer la durée de transit (A2-I), correspondant à la durée de transit entre l'endroit où est acquise l'onde acoustique et l'endroit où est acquise l'onde de pression artérielle, et
   procédé dans lequel on recale des points caractéristiques liés à des évènements physiologiques cardiaques qui leur ont donné naissance, sur le même axe temporel, ce qui permet d'avoir une empreinte cardio-vasculaire de l'individu ou réglette numérique cardiaque.

2. Procédé selon la revendication 1, dans lequel on accède à la vitesse d'onde de pouls (VOP) à partir de la durée de transit, en utilisant la formule
   VOP = D / A2-I, où D est la distance entre l'endroit où est acquise l'onde acoustique et l'endroit où est acquise l'onde de pression artérielle.

3. Procédé selon l'une des revendications précédentes, dans lequel on distingue, dans un intervalle du bruit B1, la composante mitrale de la composante tricuspide et, dans un intervalle du bruit B2, la composante aortique de la composante pulmonaire.

4. Procédé selon la revendication 3, dans lequel on réalise une extrapolation d'une première partie de la courbe de pression, on accède au point de fermeture mitrale correspondant à l'intersection entre cette droite d'extrapolation et une droite passant par le premier bruit de la courbe acoustique, on détermine la pente de cette droite d'extrapolation, on réalise une droite d'extrapolation d'une autre partie de la courbe de pression, on détermine le point d'ouverture mitrale et on accède à la durée de relaxation iso volumétrique, correspondant au temps entre l'incisure catacrote de la courbe de pression et le point d'ouverture mitrale.

5. Procédé selon l'une des revendications précédentes, dans lequel on détecte l'onde T de la courbe électrique au moyen d'une méthode par morphologie mathématique multi échelles.

6. Procédé selon l'une des revendications précédentes, dans lequel on traite la courbe acoustique en utilisant une décomposition modale empirique, on obtient différentes courbes décomposées, dites d'intérêt, à partir de cette décomposition, et on sélectionne au moins une courbe décomposée préférée ayant une énergie de signal maximale.

7. Procédé selon l'une des revendications précédentes, dans lequel on sélectionne la courbe décomposée préférée ayant une valeur minimale d'un critère d'arrêt : $R_j = 1 - [E(IMF^2 j) / E(s^2(k))]$, où $E(IMF^2 j)$ désigne l'énergie de chaque courbe décomposée obtenue par la décomposition modale empirique, pour j variant de 1 à n, n étant le nombre de courbes décomposées obtenues lors d'une décomposition modale empirique donnée, et où $E(s^2(k))$ désigne l'énergie globale du signal initial.

8. Procédé selon la revendication 7, dans lequel on garde uniquement les courbes décomposées d'intérêt qui appartiennent à une plage de fréquences prédéterminée et, de préférence, on calcule un signal utile de la courbe acoustique, correspondant à la somme des courbes décomposées d'intérêt.

9. Procédé selon la revendication 8, dans lequel on localise les premier et deuxième bruits B1 et B2 de la courbe acoustique en synchronisant le signal utile avec la courbe électrique.

10. Procédé selon l'une quelconque des revendications précédentes, dans lequel :

   - on applique la transformée de Huang Hilbert au signal utile de la courbe acoustique, ce qui permet d'obtenir l'enveloppe modale de ce signal utile,
   - on détecte les maxima de cette enveloppe modale,
   - on réalise une opération de seuillage, de façon à localiser les premier et deuxième bruits B1 et B2 de la courbe acoustique ; et
   - on détecte de préférence la probabilité de présence d'un souffle systolique ou diastolique entre les bruits B1 et B2

11. Dispositif pour la détermination d'au moins un paramètre représentatif d'une activité cardio-vasculaire, comprenant:

   - au moins un capteur acoustique (11), pour l'acquisition de ladite courbe acoustique
   - au moins un capteur de pression (12), adapté pour mesurer des vibrations d'une fréquence inférieure à 5 Hz, pour l'acquisition de ladite courbe de pression,
   - au moins un capteur électrique (13), pour l'acquisition de ladite courbe électrique,
   - des moyens de traitement (15),
   - des moyens d'affichage (4) du ou des paramètre(s) estimé(s),

   et tel que le dispositif soit configuré pour mettre en œuvre un procédé conforme à l'une quelconque des revendications précédentes.

12. Dispositif selon la revendication 11, qui comprend en outre une lyre propre à être connectée audit capteur acoustique, ladite lyre possédant un microphone et des moyens de traitement du son, propres à acquérir au moins un enregistrement numérique d'une dictée reçue par le microphone, ladite lyre possédant de préférence un support magnétique pour maintenir un accessoire ou un capteur.

13. Dispositif selon la revendication 11 ou 12, **caractérisé en ce qu'**il comprend un module (200) destiné à être posé sur le thorax d'un patient, ce module étant équipé d'un capteur central (211), formant capteur acoustique, ainsi que de trois électrodes sèches (213), réparties régulièrement à la périphérie de ce capteur central, formant capteurs électriques, qui sont reliées de manière à délivrer au moins une dérivation bipolaire.

**Patentansprüche**

1. Verfahren zur Bestimmung mindestens eines für die Herz-Kreislauf-Aktivität repräsentativen Parameters, umfassend die folgenden Schritte :

   - Erfassung einer Kurve, die eine kardiale akustische Welle oder akustische Kurve (PCG) darstellt,
   - Erfassung einer Kurve vom Mechanogramm-Typ, die eine Blutdruckwelle oder Druckkurve (CAR) darstellt, durch einen Drucksensor (12), der geeignet ist zur Messung von Schwingungen mit einer Frequenz unter 5 Hz,
   - Erfassung einer Kurve, die eine elektrische Herzwelle oder elektrische Kurve (EKG) darstellt,
   - Abgleichen der akustischen Kurve, der Druckkurve und der elektrischen Kurve, um mindestens einen Parameter abzuschätzen, der für die Herz-Kreislauf-Aktivität repräsentativ ist,
   - Anzeige des/der ermittelten Parameter(s),
   wobei mindestens ein repräsentativer Parameter eine für eine kardiovaskuläre Aktivität repräsentative Dauer ist, und in welchem Verfahren diese Dauer gemessen wird, indem als Beginn dieser Dauer ein Punkt (A2) aus einem Punkt der akustischen Kurve (PCG) und einem Punkt der Druckkurve und als Ende dieser Dauer der andere (I) aus dem Punkt der akustischen Kurve und dem Punkt der Druckkurve (CAR) genommen wird, wobei die Dauer aus zwei Punkten berechnet wird, die auf zwei Kurven unterschiedlicher Morphologie gemessen wurden, nämlich der akustischen Kurve (PCG) und der Druckkurve (CAR),
   wobei diese Messung die Bestimmung der Laufzeit (A2-I), die der Laufzeit zwischen dem Ort, an dem die Schallwelle erfasst wird, und dem Ort, an dem die arterielle Druckwelle erfasst wird, entspricht, ermöglicht, und in welchem Verfahren charakteristische Punkte, die mit den kardialen physiologischen Ereignissen verknüpft sind, die sie verursacht haben, auf derselben Zeitachse neu ausgerichtet werden, was einen kardiovaskulären Abdruck des Individuums oder des digitalen Herzlineals ermöglicht.

2. Verfahren nach Anspruch 1, wobei die Pulswellengeschwindigkeit (PWV) aus der Transitzeit unter Verwendung der Formel VOP = D / A2-I erhalten wird, wobei D die Entfernung zwischen der Stelle, an der die Schallwelle erfasst wird, und der Stelle, an der die arterielle Druckwelle erfasst wird, ist.

3. Verfahren nach einem der vorhergehenden Ansprüche, bei dem im Intervall des Rauschens B1 die Mitralkomponente von der Trikuspidalkomponente und im Intervall des Rauschens B2 die Aortenkomponente von der Pulmonalkomponente unterschieden wird.

4. Verfahren nach Anspruch 3, bei dem eine Extrapolation eines ersten Teils der Druckkurve durchgeführt wird, der Mitralverschlusspunkt ermittelt wird, der dem Schnittpunkt zwischen dieser Extrapolationslinie und einer Linie entspricht, die durch das erste Rauschen der akustischen Kurve verläuft, die Steigung dieser Extrapolationslinie bestimmt wird, eine Extrapolationslinie eines anderen Teils der Druckkurve erstellt wird, der Mitralöffnungspunkt bestimmt wird und die isovolumetrische Relaxationszeit ermittelt wird, die der Zeit zwischen der katakrotischen Kerbe der Druckkurve und dem Mitralverschlusspunkt entspricht.

5. Verfahren nach einem der vorhergehenden Ansprüche, bei dem die T-Welle der elektrischen Kurve mittels eines mehrskaligen mathematischen Morphologieverfahrens erfasst wird.

6. Verfahren nach einem der vorhergehenden Ansprüche, bei dem die akustische Kurve mittels einer empirischen modalen Zerlegung verarbeitet wird, aus dieser Zerlegung verschiedene zerlegte Kurven, sogenannte interessierende Kurven, gewonnen werden und mindestens eine bevorzugte zerlegte Kurve mit maximaler Signalenergie ausgewählt wird.

7. Verfahren nach einem der vorhergehenden Ansprüche, bei dem die bevorzugte zerlegte Kurve mit einem Mindestwert eines Abbruchkriteriums ausgewählt wird: $Rj = 1 - [E(IMF^2j) / E(s^2(k))]$, wobei $E(IMF^2j)$ die Energie jeder zerlegten Kurve bezeichnet, die durch die empirische modale Zerlegung erhalten wurde, für j von 1 bis n, wobei n die Anzahl der zerlegten Kurven ist, die während einer gegebenen empirischen modalen Zerlegung erhalten wurden, und wobei $E(s^2(k))$ die Gesamtenergie des ursprünglichen Signals bezeichnet.

8. Verfahren nach einem der vorhergehenden Ansprüche, bei dem nur die interessierenden zerlegten Kurven beibehalten werden, die zu einem vorgegebenen Frequenzbereich gehören, und bei dem vorzugsweise ein Nutzsignal der akustischen Kurve berechnet wird, das der Summe der interessierenden zerlegten Kurven entspricht.

9. Verfahren nach einem der vorhergehenden Ansprüche, bei dem das erste und zweite Rauschen B1 und B2 der akustischen Kurve durch Synchronisation des Nutzsignals mit der elektrischen Kurve lokalisiert werden.

10. Verfahren gemäß einem der vorhergehenden Ansprüche, wobei :

   - die Huang-Hilbert-Transformation auf das Nutzsignal der akustischen Kurve angewendet wird, wodurch es möglich wird, die modale Hüllkurve dieses Nutzsignals zu erhalten,
   - die Maxima dieser modalen Hüllkurve ermittelt werden,
   - eine Schwellenwertoperation durchgeführt wird, um das erste und zweite Rauschen B1 und B2 der akustischen Kurve zu lokalisieren, und
   - vorzugsweise die Wahrscheinlichkeit des Vorhandenseins eines systolischen oder diastolischen Geräusches zwischen den Tönen B1 und B2 erkannt wird.

11. Vorrichtung zur Bestimmung mindestens eines für die Herz-Kreislauf-Aktivität repräsentativen Parameters, umfassend:

   - mindestens einen akustischen Sensor (11) zur Erfassung der akustischen Kurve,
   - mindestens einen Drucksensor (12), der geeignet ist zur Messung von Schwingungen mit einer Frequenz unter 5 Hz, zur Erfassung der genannten akustischen Kurve,
   - mindestens einen elektrischen Sensor (13) zur Erfassung der elektrischen Kurve,

- Verarbeitungsmittel (15),
- Mittel zur Anzeige (4) des/der geschätzten Parameter(s),

und so, dass das Gerät so konfiguriert ist, um ein Verfahren gemäß einem der vorhergehenden Ansprüche auszuführen.

12. Vorrichtung nach Anspruch 11, die außerdem eine Lyra umfasst, die mit dem akustischen Sensor verbunden werden kann, wobei die Lyra über ein Mikrofon und Tonverarbeitungsmittel verfügt und geeignet ist, mindestens eine digitale Aufzeichnung eines vom Mikrofon empfangenen Diktats zu erfassen, wobei die Lyra vorzugsweise über eine magnetische Halterung zum Halten eines Zubehörs oder eines Sensors verfügt.

13. Vorrichtung nach Anspruch 11 oder 12, **dadurch gekennzeichnet, dass** sie ein Modul (200) umfasst, das dazu bestimmt ist, auf dem Brustkorb eines Patienten platziert zu werden, wobei dieses Modul mit einem zentralen Sensor (211) ausgestattet ist, der einen akustischen Sensor bildet, sowie mit drei trockenen Elektroden (213), die regelmäßig um den Umfang dieses zentralen Sensors verteilt sind und elektrische Sensoren bilden, die so verbunden sind, dass sie mindestens eine bipolare Ableitung liefern.


**Claims**

1. Method for determining at least one parameter representative of cardiovascular activity, comprising the following steps:

   - acquisition of a curve representative of a cardiac acoustic wave, or acoustic curve (PCG),
   - acquisition of a mechanogram-type curve, representative of a blood pressure wave, or pressure curve (CAR), by a pressure sensor (12) adapted to measure vibrations with a frequency lower than 5 Hz,
   - acquisition of a curve representative of a cardiac electrical wave, or electrical curve (ECG),
   - matching the acoustic curve, the pressure curve and the electrical curve to estimate at least one parameter representative of cardiovascular activity,
   - display of the determined parameter(s),
   at least one representative parameter being a duration (A2-I) representative of a cardiovascular activity,
   and in which method this duration is measured by taking, as the start of this duration, one (A2) from among a point of the acoustic curve (PCG) and a point of the pressure curve and, as the end of this duration, the other (I) from among the point of the acoustic curve and the point of the pressure curve (CAR), said duration being calculated from two points measured on two curves of different morphologies, namely the acoustic curve (PCG) and the pressure curve (CAR),
   said measurement allowing the determination of the transit time (A2-I), corresponding to the transit time between the location where the acoustic wave is acquired and the location where the arterial pressure wave is acquired, and
   in which method characteristic points linked to cardiac physiological events that gave rise to them are realigned on the same time axis, which allows for a cardiovascular imprint of the individual or digital cardiac ruler.

2. The method of claim 1, wherein the pulse wave velocity (PWV) is accessed from the transit time, using the formula VOP = D / A2-I, where D is the distance between the location where the acoustic wave is acquired and the location where the arterial pressure wave is acquired.

3. Method according to one of the preceding claims, in which, in the interval of the noise B1, the mitral component is distinguished from the tricuspid component and, in the interval of the noise B2, the aortic component from the pulmonary component.

4. Method according to claim 3, in which an extrapolation of a first part of the pressure curve is carried out, the mitral closure point corresponding to the intersection between this extrapolation line and a line passing through the first noise of the acoustic curve is accessed, the slope of this extrapolation line is determined, an extrapolation line of another part of the pressure curve is produced, the mitral opening point is determined and the iso-volumetric relaxation time, corresponding to the time between the catacrotic notch of the pressure curve and the mitral closure point, is accessed.

5. Method according to one of the preceding claims, in which the T wave of the electrical curve is detected by means of a multi-scale mathematical morphology method.

6. Method according to one of the preceding claims, in which the acoustic curve is processed using an empirical modal decomposition, different decomposed curves, called curves of interest, are obtained from this decomposition, and at least one preferred decomposed curve having a maximum signal energy is selected.

7. Method according to one of the preceding claims, in which the preferred decomposed curve having a minimum value of a stopping criterion is selected: $Rj = 1 - [E(IMF^2j) / E(s^2(k))]$, where $E(IMF^2j)$ denotes the energy of each decomposed curve obtained by the empirical modal decomposition, for j varying from 1 to n, n being the number of decomposed curves obtained during a given empirical modal decomposition, and where $E(s^2(k))$ denotes the overall energy of the initial signal.

8. Method according to one of the preceding claims, in which only the decomposed curves of interest which belong to a predetermined frequency range are kept, and in which preferably a useful signal of the acoustic curve is calculated, corresponding to the sum of the decomposed curves of interest.

9. Method according to any one of the preceding claims, in which the first and second noises B1 and B2 of the acoustic curve are located by synchronizing the useful signal with the electrical curve.

10. A method according to any preceding claim, wherein:

- the Huang Hilbert transform is applied to the useful signal of the acoustic curve, which makes it possible to obtain the modal envelope of this useful signal,
- maxima of this modal envelope are detected,
- a thresholding operation is carried out, in order to locate the first and second noises B1 and B2 of the acoustic curve, and
- preferably, the probability of the presence of a systolic or diastolic murmur between noise B1 and noise B2 is detected.

11. Device for for determining at least one parameter representative of cardiovascular activity, comprising:

- at least one acoustic sensor (11), for the acquisition of said acoustic curve
- at least one pressure sensor (12), for acquiring said pressure curve, adapted to measure vibrations with a frequency lower than 5 Hz,
- at least one electrical sensor (13), for the acquisition of said electrical curve,
- processing means (15),
- means of displaying (4) the estimated parameter(s),

and such that the device is configured to implement a method according to any one of the preceding claims.

12. Device according to claim 11, which further comprises a lyre suitable for being connected to said acoustic sensor, said lyre having a microphone and sound processing means, suitable for acquiring at least one digital recording of a dictation received by the microphone, said lyre preferably having a magnetic support for holding an accessory or a sensor.

13. Device according to claim 11 or 12, **characterized in that** it comprises a module (200) intended to be placed on the thorax of a patient, this module being equipped with a central sensor (21 1), forming an acoustic sensor, as well as three dry electrodes (213), distributed regularly around the periphery of this central sensor, forming electrical sensors, which are connected so as to deliver at least one bicolar derivation.

FIG. 1

FIG. 2

FIG. 3a

FIG. 4

FIG. 3b

FIG. 5

FIG. 7

FIG. 6a

FIG. 6b

Temps (s)

IMF6

Fréquence (Hz)

FIG. 6c

FIG. 11

FIG. 8

FIG. 9

FIG. 10

FIG. 12

FIG. 13

FIG. 14

FIG. 15

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2973998 A
- FR 3005256
- EP 1338242 A
- FR 2973998
- FR 2983055
- FR 2983505

**Littérature non-brevet citée dans la description**

- **LAURENT S** ; **COCKCROFT J** ; **VAN BORTEL L** ; **BOUTOUYRIE P** ; **GIANNATTASIO C** ; **HAYOZ D** ; **PANNIER B** ; **VLACHOPOULOS C** ; **WILKINSON I** ; **STRUIJKER-BOUDIER H**. European Network for Non-invasive Investigation of Large Arteries. Expert consensus document on arterial stiffness: methodological issues and clinical applications. *Eur Heart J.*, 2006, vol. 27, 2588-605
- **P. MARAGOS** ; **R. W. SCHAFTER** ; **M. A. BUTT**. Mathematical Morphology and Its Applications to Image and Signal Processing. Kluwer, 1996
- **P. E. TRAHANIAS**. An approach to QRS detection using mathematical morphology. *IEEE Trans Biomed Eng*, February 1993, vol. 40 (2), 201-205